(19) **Europäisches Patentamt**
**European Patent Office**
**Office européen des brevets**

(11) **EP 3 730 941 A1**

(12) **EUROPEAN PATENT APPLICATION**

(43) Date of publication:
**28.10.2020 Bulletin 2020/44**

(51) Int Cl.:
**G01N 33/574** (2006.01)  **G16B 20/00** (2019.01)

(21) Application number: **19305522.5**

(22) Date of filing: **23.04.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Institut Jean Paoli & Irène Calmettes**
  **13009 Marseille (FR)**
• **Ligue Nationale Contre le Cancer**
  **75013 Paris (FR)**
• **Institut National de la Santé et de la Recherche Médicale**
  **75013 Paris (FR)**
• **Université d'Aix Marseille**
  **13007 Marseille (FR)**
• **CENTRE NATIONAL DE LA RECHERCHE SCIENTIFIQUE**
  **75016 Paris (FR)**

(72) Inventors:
• **TURRINI, Olivier**
  **13007 MARSEILLE (FR)**
• **GILABERT, Marine**
  **13008 MARSEILLE (FR)**
• **GIOVANNINI, Marc**
  **13400 AUBAGNE (FR)**
• **NICOLLE, Rémy**
  **94100 SAINT-MAUR-DES-FOSSÉS (FR)**
• **BLUM, Yuna**
  **35000 RENNES (FR)**
• **IOVANNA, Juan**
  **13008 MARSEILLE (FR)**
• **DUSETTI, Nelson**
  **13008 MARSEILLE (FR)**

(74) Representative: **Novagraaf Technologies**
  **Bâtiment O2**
  **2, rue Sarah Bernhardt**
  **CS90017**
  **92665 Asnières-sur-Seine Cedex (FR)**

(54) **METHOD FOR DETERMINING A REFERENCE TUMOR AGGRESSIVENESS MOLECULAR GRADIENT FOR A PANCREATIC DUCTAL ADENOCARCINOMA**

(57)    The present invention relates to a method for determining a reference tumor aggressiveness molecular gradient for at least one pancreatic ductal adenocarcinoma (PDAC) sample, to a method for determining a specific tumor aggressiveness molecular gradient from a plurality of particular PDAC samples, and to methods for determining a tumor aggressiveness of a PDAC sample to be diagnosed. The method for determining a reference tumor aggressiveness molecular gradient comprises: generating a first matrix of transcriptomic profiles from first vectors representing respectively reference transcriptomic profiles of tumor samples of human or animal origin; obtaining, for each reference transcriptomic profile, a tumor aggressiveness histological class; decomposing the first matrix by a matrix factorization method allowing an identification of at least partially decorrelated components; identifying a first component that is the most discriminating relatively to the tumor aggressiveness histological classes associated with the reference transcriptomic profiles; determining the reference tumor aggressiveness molecular gradient as a distribution of projections of the first vectors on the first component.

Obtain a matrix of transcriptomic profiles from first vectors representing respectively reference transcriptomic profiles of tumor samples — 200

Obtaining, for each reference transcriptomic profile, a tumor aggressiveness histological class — 210

Decomposing the matrix by a matrix factorization method allowing an identification of at least partially decorrelated components — 220

Identifying a first component that is the most discriminating relatively to the tumor aggressiveness histological classes associated with the reference transcriptomic profiles — 230

Determining the reference tumor aggressiveness molecular gradient as a distribution of projection values of the first vectors on the first component — 240

**Figure 2**

## Description

### Technical field

The present invention relates to a method for determining a reference tumor aggressiveness molecular gradient for at least one pancreatic ductal adenocarcinoma (PDAC) sample, to a method for determining a specific tumor aggressiveness molecular gradient from a plurality of particular PDAC samples, and to methods for determining a tumor aggressiveness of a PDAC sample to be diagnosed.

The invention relates to the field of transcriptomics, and effective diagnosis and follow-up during the treatment of individuals with a Pancreatic Ductal Adenocarcinoma (PDAC). It also relates methods and algorithms suitable for use in determining the prognosis of a patient with PDAC, based on its predicted histology. The present invention provides notably an effective support to the physician to classify a PDAC and select the most efficient anticancer compound for each PDAC patient.

In the present document, the numbers between brackets ([ ]) refer to the List of References provided at the end of the document.

### Background of the Invention

Pancreatic Ductal Adenocarcinoma (PDAC) is one of the most aggressive gastrointestinal tumors. An earliest genetic event in the progression of the normal ductal epithelia to premalignant pancreatic intraepithelial neoplasia (PanIN) is the mutation of the K-Ras oncogene (Deramaudt T, Rustgi AK, Mutant KRAS in the initiation of pancreatic cancer, Biochim Biophys Acta. 2005 Nov 25; 1756(2):97-101 [1]). The mutational activation of KRAS protein triggers diverse downstream effector proteins that sustain proliferation, metabolic reprogramming, anti-apoptosis, evasion of the immune response and remodeling of the microenvironment. Subsequently, chromosome rearrangements and mutations in CDKN2A, TP53, SMAD4 and other genes cause the progression from low- to high-grade PanIN and lead to PDAC (Feldmann et al., 2007 [2]; Iovanna et al., 2012 [3]; Ying et al., 2016 [4]). Tumor mass contains also cells such as the cancer-associated fibroblasts (CAFs), the T cells, the stellate cells, the macrophages, the regulatory T cells, the endothelial cells and others (Ryan Carr et al. 2016 [5]; and Pillarisetty, 2014 [6]). They play an active role in maintaining a favorable microenvironment for cancer cell survival. At molecular level, the coordination among stromal cells and between cancer and stromal cells is sustained by the exchanges of molecules (Iovanna and Closa, 2017 [7]; Leca, J. et al. JCI. 126, 1-17, 2016 [8]).

PDAC is a heterogeneous disease with a variable clinical evolution, an inconstant response to the treatments and histopathologically may present some differentiation degrees. In fact, survival after the time of diagnosis could be from 3 months to more than 5-6 years or even more, efficient response to the gemcitabine treatments is of 10% (Burris et al., 1997 [9]) whereas to folfirinox is around 30% (Conroy et al., 2011 [10]), and histologically PDAC tumors could be from very to poorly differentiate. We (Nicolle et al., 2017 [11]) and others (Bailey et al., 2016 [12]) have reported that the clinical evolution, the response to the treatments as well as the differentiation degrees cannot be explained by the genetic mutations. On the contrary, we demonstrated that at least the overall survival and histological characteristics of the tumors are determined by the epigenetic landscape including DNA methylation (Nicolle et al., 2017 [11]) and specific histones marks (Lomberk et al., 2018 [13]). This is conceptually important since epigenetic, contrary to the majority of the genetic mutation, is druggable and therefore modifiable.

Predicting the clinical evolution and outcome of PDAC at the diagnosis time is of evident clinical interest, in particular for selecting an appropriate anticancer treatment.

Histology is a good indicator to predicting the survival time (Lenz et al., 2011 [14]) but is it only possible in a small amount as lesser than 15% of the operable patients. While on the non-operable patients, who have a most evolved disease and represent around of 85%, the only available material is a small number of fresh cells, obtained by Endoscopic ultrasound-guided fine needle aspiration biopsy (EUS-FNA), that results not enough for determine its differentiation status. Therefore to phenotypically characterize all the PDAC became urgent since it could be determinant for the therapeutic approach. In particular, there remains a need for *in vitro* or *ex vivo* methods for determining the short-term and long-term survival (prognosis) of said individual; or alternatively for determining the level of differentiation of PDAC in said individual.

There are still no available prognostic biomarkers, or combinations of biomarkers for patients with PDAC; nor biomarkers of predictive response. Clinical heterogeneity is one of the main features of Pancreatic Adenocarcinoma (PDAC). Conventional clinico-pathological analyses such as grading are only achievable for the 15% of resectable patients and provide limited value.

Therefore, a more robust and clinically useful prognostic indicator applicable to all PDAC sample is required. There remains a need for in vitro or ex vivo methods for determining the prognosis of a patient with PDAC, providing an effective support for the physician for the selection of an appropriate drug for treating these patients, which includes therapeutic treatments with active principles such as gemcitabine, oxaliplatin, 5-fluorouracil (5FU), docetaxel, Irinotecan, and/or derivatives thereof.

**Description of the invention**

It is an object of the invention to provide improved *in vitro* or *ex vivo* methods for the diagnosis and prognosis of pancreatic ductal adenocarcinoma (PDAC), in particular for determining the differentiation status of the PDAC.

According to a first aspect, the present invention relates to a method for determining a reference tumor aggressiveness molecular gradient for at least one pancreatic ductal adenocarcinoma (PDAC) sample, the method comprising:

- generating a first matrix of transcriptomic profiles from first vectors representing respectively reference transcriptomic profiles of tumor samples of human or animal origin;
- obtaining, for each reference transcriptomic profile, a tumor aggressiveness histological class;
- decomposing the first matrix by a matrix factorization method allowing an identification of at least partially decorrelated components;
- identifying a first component that is the most discriminating relatively to the tumor aggressiveness histological classes associated with the reference transcriptomic profiles;
- determining the reference tumor aggressiveness molecular gradient as a distribution of projection values of the first vectors on the first component, wherein the orientation of the scale of the projection values is determined on the basis of the correspondence between at least one tumor aggressiveness histological class and the distribution of the projection values of the first vectors representing reference transcriptomic profiles belonging to said at least one tumor aggressiveness histological class.

According to a second aspect, the present invention relates also to a method for determining a tumor aggressiveness of a PDAC sample to be diagnosed, the method comprising:

- obtaining a vector representing a transcriptomic profile of the PDAC sample to be diagnosed;
- obtaining a reference tumor aggressiveness molecular gradient by the method according to the first aspect;
- computing a score representative of the tumor aggressiveness of the PDAC sample as the projection value of the vector representing the transcriptomic profile of the PDAC sample on the first component for which the reference tumor aggressiveness molecular gradient has been obtained; and
- comparing the score with the reference tumor aggressiveness molecular gradient.

According to a third aspect, the present invention relates also to a method for determining a specific tumor aggressiveness molecular gradient from a plurality of PDAC samples, the method comprising:

- generating a second matrix of transcriptomic profiles from second vectors representing respectively specific transcriptomic profiles of the plurality of PDAC samples;
- decomposing the second matrix by a matrix factorization method allowing an identification of at least partially decorrelated components;
- determining a first component and a reference tumor aggressiveness molecular gradient by the method according to the first aspect;
- identifying a second component that is the most statistically associated with the first component for which the reference tumor aggressiveness molecular gradient has been obtained;
- determining the specific tumor aggressiveness molecular gradient as a distribution of projection values of the second vectors on the second component, wherein the orientation of the scale of the projection values is determined on the basis of the correspondence between the reference tumor aggressiveness molecular gradient and the distribution of the projection values of the second vectors on the second component.

The advantage of this method, which makes it possible to obtain a specific gradient, is that it does not require further histological classification experiments. Indeed, according to this method, the specific gradient can be deduced from a reference gradient determined according to the invention's method.

Accordingly, advantageously based on an available reference gradient determined according to the present invention, the present invention allows the determination of a tumor aggressiveness of a PDAC sample to be diagnosed without the need of further histological classification experiments.

Thus, according to a fourth aspect, the present invention relates also to a method for determining tumor aggressiveness of a PDAC sample to be diagnosed, the method comprising:

- obtaining a vector representing a transcriptomic profile of the PDAC sample to be diagnosed;
- obtaining a second component and a specific tumor aggressiveness molecular gradient by a method according to the third aspect;
- computing a score representative of the tumor aggressiveness of the PDAC sample as the projection value of the vector representing the transcriptomic profile of the PDAC sample on the second component for which the specific tumor aggressiveness molecular gradient has been obtained; and
- comparing the score with the specific tumor aggressiveness molecular gradient.

The present invention relates also to a computer readable medium comprising computer program instructions which when executed causes an apparatus to perform the steps of a method according to any of the first, second, third or fourth aspects as defined and described herein.

The present invention relates also to an apparatus comprising means for performing the steps of a method according to any of the first, second, third or fourth aspects as defined and described herein.

As used herein, the term "sample" or "tumor sample" means any tissue tumor sample derived from a patient. Said tissue sample is obtained for the purpose of the in vitro evaluation. The sample can be fresh, frozen, fixed or embedded (e.g., paraffin embedded) or derived (Patients-derived Tumor Xenografts (PDTX)). The tumor sample may result from the tumor resected from the patient. The tumor sample may also result from a biopsy performed in the primary tumor of the patient or perfomed in metastatic sample distant from the primary tumor of the patient. The sample may be obtained for example through endoscopic ultrasound-guided fine-needle aspiration (EUS-FNA) biopsy samples from patients with unresectable tumors or from tumor tissue samples from patients undergoing surgery as disclosed in Vilmann P et al. 1992 [15].

When the samples are Patients-derived Tumor Xenografts (PDTX), the samples may be obtained by any protocol known by the skilled person, for example by the protocol disclosed in document Duconseil et al., 2015 [16]. The same applies for a sample to be diagnosed by the methods of the present invention.

According to the present invention, most preferably, the method for preparing the sample to be diagnosed is the same as the method used for the determination of the reference or specific gradient.

As used herein, the term "patient" means human or animal patient.

As used herein, the term "gradient" is intended to refer to a distribution of values defined with respect to a graded and oriented scale representing a given physical variable, here aggressiveness. In the present invention, it is intended to refer to a distribution of projection values of the first vectors on a first component resulting from a matrix factorization of a matrix of transcriptomic profiles. This gradient allows to determine the differentiation status of the PDAC and particularly the aggressiveness of a tumor sample and is referred to therein as "tumor aggressiveness molecular gradient" or, for the sake of brevity, "molecular gradient".

Different types of molecular gradient may be computed: reference molecular gradient and specific molecular gradient.

For a reference molecular gradient determined from reference transcriptomic profiles for which histological classes are known, the orientation of the scale of the projection values of the distribution is determined on the basis of the correspondence between at least one tumor aggressiveness histological class and the distribution of the projection values of the first vectors representing reference transcriptomic profiles belonging to said at least one tumor aggressiveness histological class.

According to the present invention, in the step of determining the reference tumor aggressiveness molecular gradient as a distribution of projections of the first vectors on the first component, the orientation of projection value scale of the distribution may advantageously be determined based on the correspondence between at least one tumor aggressiveness histological class representing the highest and / or lowest aggressiveness and a distribution of projections of first vectors representing reference transcriptomic profiles belonging to said at least one tumor aggressiveness histological class.

For a specific molecular gradient determined from specific transcriptomic profiles obtained using a specific technology, a reference molecular gradient is determined and the orientation of the scale of the projection values is determined on the basis of the correspondence between the reference molecular gradient and the distribution of the projections of the second vectors on the second component resulting from a matrix factorization of a second matrix representing the specific transcriptomic profiles.

According to the present invention, the first vectors representing respectively reference transcriptomic profiles of tumor samples of human or animal origin may be obtained by any method known by the skilled person. The methods described below are not specific to any gene expression quantification method and can be applied to any RNA-based quantification of gene expression without any specificity on processing and normalization, which includes but is not limited to: RT-qPCR, NanoString, short read Illumina sequencing often referred to as RNA-Seq, long read sequencing, microarray-technology, etc. For example, the following publications are disclosing such methods:

1. Nanostring: Kulkarni MM. Digital multiplexed gene expression analysis using the NanoString nCounter system. Curr Protoc Mol Biol. 2011 Apr;Chapter 25:Unit25B.10. doi:10.1002/0471142727.mb25b10s94. PubMed PMID: 21472696 **[17]**

2. RNA-seq and microarrays: Marioni JC, Mason CE, Mane SM, Stephens M, Gilad Y. RNA-seq: an assessment of technical reproducibility and comparison with gene expression arrays. Genome Res. 2008 Sep;18(9):1509-17. doi: 10.1101/gr.079558.108. Epub 2008 Jun 11. PubMedPMID: 18550803; PubMed Central PMCID: PMC2527709 **[18]**.

3. RNA-seq: Wang Z, Gerstein M, Snyder M. RNA-Seq: a revolutionary tool for transcriptomics. Nat Rev Genet. 2009 Jan;10(1):57-63. doi: 10.1038/nrg2484. Review. PubMed PMID: 19015660; PubMed Central PMCID: PMC2949280 **[19]**.

4. Steinbock LJ, Radenovic A. The emergence of nanopores in next-generation sequencing. Nanotechnology. 2015; 26(7):074003. doi: 10.1088/0957-4484/26/7/074003. Epub 2015 Feb 2 **[20]**

The first vectors may be obtained by collecting for each sample the level of expression of each measured gene by obtaining the relative quantification of the corresponding mRNAs. Each gene-associated mRNA quantification, a real value, is concatenated to form a vector of real values.

According to the methods of the present invention, the reference transcriptomic profiles of tumor samples of human or animal origin for the determination of the reference or specific gradient may advantageously be obtained from patients-derived tumor xenografts.

According to the methods of the present invention, the transcriptomic profiles may for example be based on RNA expression of REG4, CTSE, CLDN18, TM4SF4, UGT2A3, SLC4A4, ANXA10, DPCR1, MUC17, HEPH, LYZ, TSPAN8 and TFF1 genes.

According to the present invention, in the method for determining tumor aggressiveness of a PDAC sample to be diagnosed based on a reference gradient, the vector representing a transcriptomic profile of the PDAC sample to be diagnosed is most preferably obtained following the same process as the one used for obtaining the reference gradient.

According to the present invention, in the method for determining tumor aggressiveness of a PDAC sample to be diagnosed based on a specific gradient, the vector representing a transcriptomic profile of the PDAC sample to be diagnosed is most preferably obtained following the same process as the one used for obtaining the specific gradient.

Advantageously, however, the method used for obtaining the vectors representing respectively the reference transcriptomic profiles of tumor samples of human or animal origin needs not to be the same as the one used for obtaining the vectors representing respectively the specific transcriptomic profiles of tumor samples of human or animal origin for the determination of the specific gradient.

According to the present invention, for the determination of the reference gradient, the tumor aggressiveness histological classes are obtained from the same patients-derived tumor xenografts samples as those used to obtain the reference transcriptomic profiles, for example from patients-derived tumor xenografts. The tumor aggressiveness histological classes may be defined for example on the basis of microscopic observations of the cells of the samples. In the experiments disclosed below, the inventors have advantageously defined a criterion for histological classification able to class the PDTX into five classes: from class I to V, respectively from the most to the less aggressive tumor cells. Any other suitable classification, even with more or less classes, preferably more, may be used according to the present invention, provided that it allows to determine a reliable aggressiveness reference gradient. This is the case with the five classes defined by the inventors in their experiments.

According to the present invention, the most discriminating component may for example be identified by evaluating a statistical association between the distribution of the projections of the first vectors on the at least partially decorrelated components and the tumor aggressiveness histological classes.

According to the present invention, the statistical association may for example be performed using an algorithm based on an analysis of variance, linear models, generalized linear models, a Kruskal-Wallis test or a Pearson or Spearman correlation.

According to the present invention, the methods for determining tumor aggressiveness of a PDAC sample to be diagnosed may further comprise the step of computing a normalized score from the score and the mean and standard deviation of the specific tumor aggressiveness molecular gradient.

According to the present invention, when the method is carried out based on a reference aggressiveness gradient, the identification of the second component may for example be performed by searching a correlation between the projections of the second vectors on the at least partially decorrelated components resulting from the matrix factorization of the second matrix and the projections of the second vectors on the first component.

According to the present invention, when the method is carried out based on a reference aggressiveness gradient, the identification of the second component may for example be performed by searching a correlation between the vectors of the coefficient matrix resulting from the matrix factorization of the second matrix and the vector of the coefficient matrix resulting from the matrix factorization of the first matrix corresponding to the first component.

According to the methods of the present invention, the matrix factorization method may be selected among an independent component analysis (ICA) method, a non-negative matrix factorization method, a principal component analysis (PCA) method, a multi-dimensional scaling (MDS) method, a matrix decomposition based on eigenvalues method, a correspondence (CA) analysis method, a latent semantic analysis (LSA) method, a canonical correlation method, a factor analysis method.

According to the methods of the present invention, the matrix factorization method may be an independent component analysis and the first component may be the first independent component that is the most discriminating relatively to the tumor aggressiveness histological classes associated with the reference transcriptomic profiles.

The inventors developed a molecular and computational analysis to predict the survival of patients with PDAC by describing its molecular grade. The methods of the present invention can be applied to predict the clinical outcome to virtually any patients with PDAC from which it is possible to obtain few cancerous cells.

Other features and advantages of the invention will be apparent from the following examples, which contain additional information, exemplification and guidance that can be adapted to the practice of this invention in its various embodiments and the equivalents thereof. These examples should not be interpreted in any way as limiting the scope of the present invention.

## Brief description of the figures

- **Figure 1a** shows five different histological cells sections pictures observed under light microscope NIKON Eclipse 90i, illustrating the experimental results on the histological classification disclosed in example 1 below.
- **Figure 1b** shows patients Overall Survival (OS) curves for the 5 different PDAC-derived PDX histopathology classes I to V.
- **Figure 1c** is an illustrative contingency table between patient operability status and the histopathology class as observed in their corresponding PDX, for classes I to V.
- **Figure 2** shows a flowchart of a method for determining a reference molecular gradient.
- **Figure 3** shows a flowchart of a method for determining a tumor aggressiveness of a PDAC sample from a reference molecular gradient.
- **Figure 4** shows a flowchart of a method for determining a specific molecular gradient.
- **Figure 5** shows a flowchart of a method for determining a tumor aggressiveness of a PDAC sample from a specific molecular gradient.
- **Figures 6a-6c** show the association between the molecular gradient and the PDX histological classes.
- **Figures 7a-7d** show prognostic value of the molecular grading system in ICGC series of resected PDAC analysed by microarray.
- **Figures 8a-8d** show prognostic value of the molecular grading system in ICGC series of resected PDAC analysed by RNA-seq technology.

## Examples

In the different experimentations carried out, the inventors have defined the phenotype of an important number of Patients-derived Tumor Xenografts (PDTX), represented all the PDAC phenotypes, histologically classified the PDTX cells, obtained their whole transcriptome, identified an RNA signature associated to each histological class by using an Independent Component Analysis (ICA) statistical method, and determined a reference tumor aggressiveness molecular gradient. The inventors have also identified a group of genes significantly associated to a given phenotype to be used as molecular markers. Patient informed consent forms were collected and registered in a central database.

## Example 1: Histology classification

Starting from 76 different patients, as part of the PaCaOmics clinical protocol under the Paoli Calmettes Institute clinical trial "Predictive Biomarkers of Therapeutic Response in Pancreatic Tumors", multicentric clinical trial NCT01692873, 76 different samples of PDAC tumor cells have been obtained. All patients presented a suspicion of PDAC and were included in the PaCaOmics clinical trial promoted by the Paoli-Calmettes Institute.

The tumor tissue used for xenograft development was deemed excess to that required for the patient's diagnosis. Two types of samples were obtained: endoscopic ultrasound-guided fine-needle aspiration (EUS-FNA) biopsy samples from patients with unresectable tumors and tumor tissue samples from patients undergoing surgery as disclosed in Vilmann P et al. 1992 **[15]**, to cover the disease variation as large as possible. All the samples were anonymized, and postsurgical anatomopathology reports were provided for specimens from each patient.

By carrying out the protocol disclosed in document Duconseil et al., 2015 [16]), each sample obtained from EUS-FNA was mixed with 100 μL of Matrigel (BD Biosciences, Franklin Lakes, NJ) and was injected in the upper right flank of a nude immunosuppressed nude mice (Swiss Nude Mouse Crl: NU(lco)-Foxnlnu; Charles River Laboratories, Wilmington, MA). Each sample derived from surgery resection was fragmented, mixed with 100 μL of Matrigel, and implanted with a trocar (10 gauge; Innovative Research of America, Sarasota, FL) in the subcutaneous right upper flank of an anesthetized and disinfected mouse.

When the tumors reached 1 cm$^3$, the mice were sacrificed, and the tumors were removed. Xenografts that failed to develop within 6 months were discontinued. The study on animals was approved by the Animal Facility and Experimental Platform (Scientific and Technological Park of Luminy, Marseille, France). After no more than 2 or 3 passages in mice (re-implantation into new mice), we have defined as precisely as possible the level of tumor differentiation from small pieces of the obtained PDTX.

Histopathologic evaluation was performed on 5-μm hematoxylin and eosin-stained sections of xenografts and examined under a light microscope NIKON Eclipse 90i. Based on this cells material, we have defined a criterion for histological classification able to class the PDTX into 5 classes: class I to V from the most to the less aggressive tumor cells. In our cohort, we first observed 3 main degrees of differentiation namely poorly, moderately and well differentiated, representing 17%, 16% and 67% of the cohort size, respectively. However, in the poorly differentiated PDTX, regarding nuclear characteristics, 62% show high atypia (Class I) and 38% low atypia (Class II). Hundred percent of the moderately differentiated PDTX show high atypia (Class III). The well differentiated PDTX shows heterogeneity regarding cytoplasmic characteristics. Around 75% of well differentiated PDTX exhibit cubical cells (Class IV) whereas 25% of them showed cylindrical cells (Class V).

In detail, tumors were considered low differentiated when tissue architecture is solid, forming massive structures or with isolated cells without visible glandular structures. Within this group, we distinguished the two different classes I and II accordingly to the degree of cyto-nuclear atypia and degree of mitosis. Class I includes tumors where nucleo-cytopasmic ratio is high (> 0.5), nuclei with irregular contours, dense chromatin, or prominent nucleolus. High proportion of mitoses was also visible in this subgroup. Class II include tumors with less atypia with a nucleo-cytoplasmic ratio < 0.5, regular-contoured nuclei, fine chromatin and a fine nucleolus. Mitoses were less frequent than in class I. Class III includes tumors that were moderately differentiated with both tissue architectures, glands and massive structures and nucleo-cytoplasmic atypia were less frequent than in class I and II.

Well differentiated PDX present a glandular architecture without solid component. In this group, we also distinguished the two subclasses IV and V accordingly to their cytological characteristics. In fact, class IV presents glands with cubic or short cylindrical cells with low or absent mucus secretion. The nuclei remain predominantly polarized and the atypies are a more marked than in the group V (looser chromatin, increase in the size of the nuclei when compared with class V). Mitoses were more frequent than in class V. Class V was the most differentiated group, the glands secrete mucin and cells present a cylindrical form, the nucleus was localized at the basal pole of the cell (polarized). Nuclei were small, with regular contours and mature chromatin without visible nucleolus. Mitoses were less frequent that in class IV.

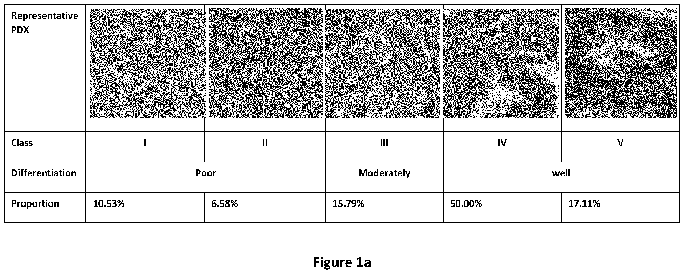

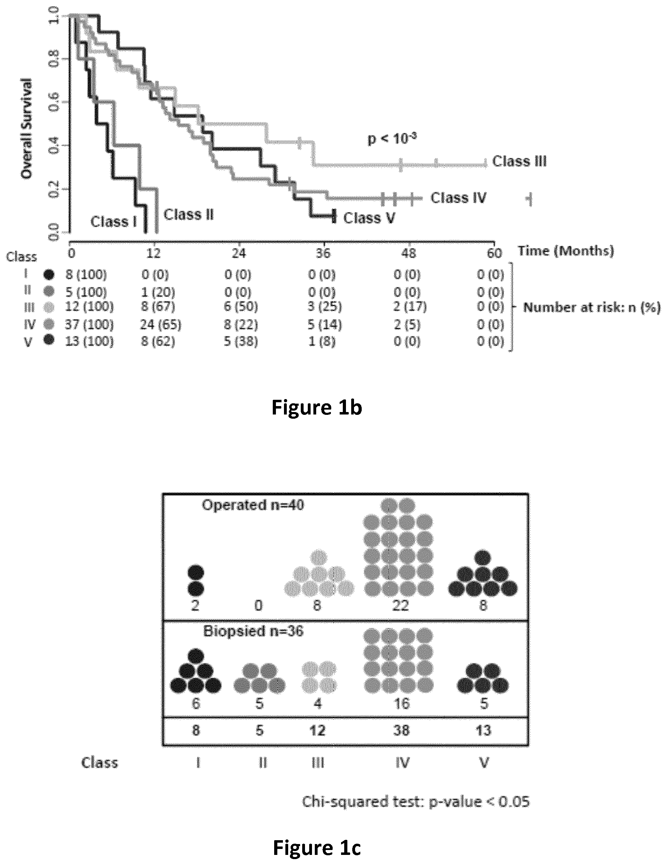

Annexed figure 1 presents five different histologic cells sections pictures obtained through these experimentations, observed under the light microscope NIKON Eclipse 90i, illustrating the resulting histological classification.

Patient cohort

Between February 2011 and September 2015 seventy-six patients whose PDAC diagnosis was confirmed were included in this study. Tumor samples were obtained from pancreatectomy in 40 patients (52.6%), EUS-FNA in 25 patients (32.9%) and carcinomatosis or liver metastasis during explorative laparotomy in 11 patients (14.5%). Their characteristics are summarized in Table I and detailed in Supplementary Table I.

Defining the histological grade of PDAC-derived PDX

**Figs. 1a-1c** illustrate histological classification of PDAC-derived PDX in which:

- **Fig. 1a** is a table showing a representative examples for each of the 5 histological classes I to V and the associated proportions within the poor, moderate- and well-differentiated PDX in the PaCaOmics cohort (total n=76);
- **Fig. 1b** shows patients OS curves for the 5 different PDAC-derived PDX histopathology classes I to V;
- **Fig. 1c** is an illustrative contingency table between patient operability status and the histopathology class as observed in their corresponding PDX.

The PDX-based 5-classes classification of PDAC was associated with the resectability of patient at diagnosis. 11 of 13 of class I and II (85%) patients were not eligible for upfront surgery (**Fig. 1b**). The PDX-based 5-classes were associated with OS of the patients from which they were derived (surgically-independent score test p-value <0.001).

We conclude that these results show the high capacity of the histological characterization of PDX to predict the clinical evolution of patients.

**Example 2:** obtention of first vectors representing respectively reference transcriptomic profiles of the tumor samples

Starting from each of the 76 pancreatic PDTX obtained from example 1, we have then performed the RNA-seq protocol using trizol and Guanidine Isothiocyanate and isolated with the miRneasy Mini kit (Qiagen). RNA-seq as disclosed in Lomberk et al., 2018 [13] and in Nicolle et al., 2017 [11].

Briefly, RNA expression profiles were obtained using Illumina's TrueSeq Stranded mRNA LT protocol. Sequencing followed oligo-dT capture and was done on a paired-end 100 pair flow cell. RNA libraries were prepared and sequenced by AROS Applied Biotechnology A/S (Aarhus, Denmark). RNA-seq reads were mapped using STAR 18 with the proposed ENCODE parameters, as disclosed in Dobin A, Davis CA, Schlesinger F, Drenkow J, Zaleski C, Jha S, Batut P, Chaisson M, Gingeras TR. STAR: ultrafast universal RNA-seq aligner, Bioinformatics, 2013 [21] Jan 1;29(1):15-21. doi: 10.1093/bioinformatics/bts635. Epub 2012 Oct 25. PubMed PMID: 23104886; PubMed Central PMCID: PMC3530905 [22].
and SMAP on the human hg19 genome and transcript annotation (Ensembl 75), and mouse mmu38 genomes.

Gene expression profiles were obtained using FeatureCounts, for assigning sequence reads to genomic features, as disclosed in Liao Y, Smyth GK and Shi W. featureCounts: an efficient general-purpose program for assigning sequence reads to genomic features. Bioinformatics, 30(7):923-30, 2014 [23]. Only genes with at least three read counts in at least 3 samples were kept for further analysis. Gene counts were normalized using the upper-quartile approach as disclosed in Bullard et al., 2010 [23].

The next step was to identify an RNA signature associated to each histological class since we demonstrated that the phenotype of the PDAC is controlled by the transcriptome and epigenome (Lomberk et al., 2018 [13]; Nicolle et al., 2017 [11]). We used the Independent Component Analysis (ICA) statistical method to define in a semi-supervised manner a gene expression component that best correlated with the histological classification. The component 3, noted ICA3, from an ICA with 3 components in total, was the component with the most significant association with the histological classification (p < 10-3). The other ICAs (1 and 2) are not significantly associated with the class of differentiation. We conclude that the component 3, of the ICA, which is defining a complex RNA signature, is significantly associated to the histology of the PDAC. These data is clinically relevant since the transcriptome is reflecting the histology of the PDAC. Then, we selected the minimal number of genes associated to the ICA3 which we named "minimal ICA3" (see signature 0 on the annex 1).

Then, we analyzed the effect of the minimal ICA3 signature on the overall survival. We hypothesized that if histology is associated to a clinical outcome and in turn the transcriptome is associated to the histology, therefore the transcriptome should be associated to the clinical outcome. The first finding controlling the overall survival is mainly driven by the surgery factor as expected. Indeed, the operation reduces the hazard by 76% (p < 10-3). Using a stratified Cox model (strata= operated and biopsied), we observed that the histopathological classes tended to explain overall survival (p = 0.09). Modeling overall survival showed also that the minimal ICA3 was the best explanatory factor. Indeed, the stratified model for surgery factor did not show any significant effect of ICA1 and ICA2 on the overall survival (p > 0.05). Conversely, minimal ICA3 signature is associated with a reduction of the hazard by 65%. We additionally performed cox proportional hazard model either within operated group or within biopsied group. Whereas within the operated group the minimal ICA3 was significantly associated with the overall survival (p < 0.05), within the biopsied group, the overall survival, tend to be associated to minimal ICA3 (p = 0.05). Taken together, we can affirm that the minimal ICA3 is associated to the histological classes of PDAC but more important it is associated to the overall survival.

Validation of the signature on an independent cohort of PDAC: ICGC is a public cohort of PDAC presenting two independent platforms of RNA expression analysis named microarrays and RNA-seq. ICGC cohort contains 269 patients all with resected pancreatic tumors as disclosed in Nicolle et al, 2017 [11] and Bailey et al. [12].

This cohort includes clinicopathological- and follow-up data with genome-wide expression profiling done in two platforms of RNA expression analysis namely microarrays (n=269) and RNA-seq (n=92), more precisely 76 patients were overlapped between RNAseq and Microarray datasets.

This is important because we can control the methodology-dependence of our approach. Data on overall survival and histology is also available but restricted to well-, moderately- and poor-differentiated. We applied the same approach as for PaCaOmics data and selected an ICA equivalent to the ICA3. We then analyzed independently their correlations with the overall survival. The best matching pairs of ICA3 in ICGC microarrays and RNA-seq platforms were the ICA2s of the K-systems 5. There were 6557 and 15656 common annotated genes between our PaCaOmics cohort and ICGC in microarrays and RNA-seq platforms respectively. The correlations (absolute value of spearman's rho coefficient) between ICA3 and the ICA2s from microarrays and RNA-seq platforms were 0.61 and 0.66 respectively. Using the univariate Cox model, the ICA2s from both platforms were significantly associated with overall survival (p < 0.01). As observed in the reference data set (PaCaOmics cohort) the ICA2s were significantly differential between histological classes. The multivariate models showed that ICA2s from both platforms adjusted for tumor class were significantly associated to overall survival.

The available data on OS and histology is restricted to well-, moderate- and poor-differentiated in Microarray's sample set and in moderately- and poorly-differentiated in RNA-seq sample set. Their transcriptomic profiles were assessed with Affymetrix® microarrays technology. The molecular grades of patient were classified in well-, moderate- and poor-differentiated as well.

A representative gene signature deduced from these experiments is the following one: REG4; CTSE; CLDN18; TM4SF4; UGT2A3; SLC4A4; ANXA10; DPCR1; MUC17; HEPH; LYZ; TSPAN8; TFF1. This signature is a basis to define an aggressiveness gradient according to the process of the present invention. It does not limit the scope of the present invention since some of these genes may be replaced or other genes may be added to this signature. This signature represents however a confident basis for carrying out the present invention.

**Example 3:** Determination of the tumor aggressiveness molecular gradient

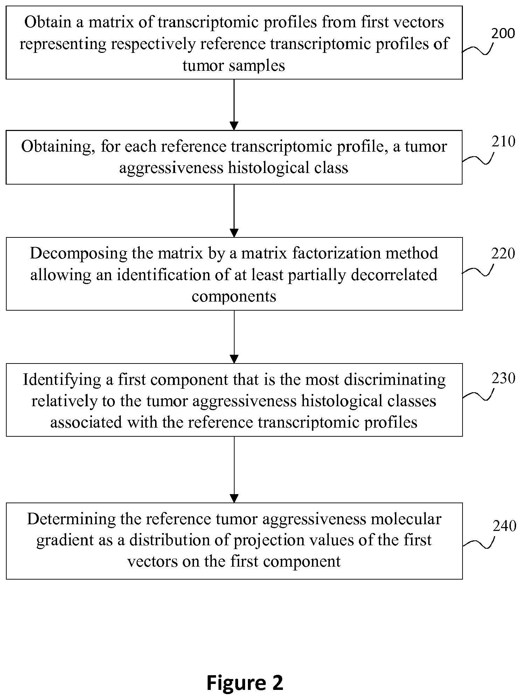

A statistical approach will be described in details below by reference to figures 2 to 5.

Example embodiments of the statistical analysis will now be described below with reference to functions, engines, block diagrams and flowchart illustrations of the methods, systems, apparatus and computer program according to one or more exemplary embodiments. Each described function, engine, block of the block diagrams and flowchart illustrations can be implemented in hardware, software, firmware, middleware, microcode, or any suitable combination thereof.

If implemented in software, the functions, engines, blocks of the block diagrams and/or flowchart illustrations can be implemented by computer program instructions, which may be stored or transmitted over a computer-readable medium and executed by a general purpose computer, special purpose computer or other programmable processing apparatus and / or system, such that the computer program instructions cause the performance of the functions, engines, blocks described herein.

Reference molecular gradient

A method for determining a reference molecular gradient for one or more pancreatic ductal adenocarcinoma (PDAC) samples will now be described by reference to **Fig. 2**. The reference molecular gradient is also referred to herein as the reference tumor aggressiveness molecular gradient.

While the steps are described in a sequential manner, the man skilled in the art will appreciate that some steps may be omitted, combined, performed in different order and / or in parallel.

The reference molecular gradient is determined on the basis of a reference data set obtained from reference transcriptomic profiles generated for tumor samples. The reference transcriptomic profiles may be generated as explained in the present document or in any other manner.

We assume here that, for each reference transcriptomic profile, a tumor aggressiveness histological class is known. For example the tumor aggressiveness histological classes for a tumor sample of human origin may be obtained from patients-derived tumor xenografts.

In step 200, a matrix of transcriptomic profiles is generated from first vectors representing respectively the reference transcriptomic profiles of tumor samples. The samples may be of human or animal origin.

Each transcriptomic profile may be encoded as a data vector representing the gene expression, e.g. according to a RNA analysis of the tumor sample. The gene expression is determined with respect to a given set of genes. Each vector coefficient of a data vector representing a transcriptomic profile is equal to the number of genes obtained for a given gene of the set of genes.

Let $N1$ be the number of reference transcriptomic profiles and G be the number of genes in the set of genes for which the RNA expression was measured. Then each transcriptomic profile $i$ ($1 \leq i \leq N1$) is encoded by a vector $V1_i$ of size G*1 that may be defined as:

$$V1i = \begin{bmatrix} x1_{i,1} \\ \vdots \\ x1_{i,G} \end{bmatrix} \qquad [Eq21]$$

where j is the gene subscript varying between 1 and G.

From the N1 vectors (hereafter the reference vectors) representing the N1 reference transcriptomic profiles, a matrix M1 of vectors is generated. The size of the matrix M1 is G*N1 and the matrix M1 may be defined

$$M1 = \begin{bmatrix} x1_{1,1} & \cdots & x1_{N1,1} \\ \vdots & \ddots & \vdots \\ x1_{1,G} & \cdots & x1_{N1,G} \end{bmatrix} \qquad [Eq22]$$

In step 210, a tumor aggressiveness histological class is obtained for each reference transcriptomic profile among a plurality of tumor aggressiveness histological classes. For example, the five tumor aggressiveness histological classes I to V described by reference to **Fig. 1a** may be used.

In step 220, the matrix M1 (hereafter the reference matrix or first matrix) is then factorized using a matrix factorization method allowing an identification of at least partially decorrelated components.

Several matrix factorization methods may be used, for example an independent component analysis (ICA) method, a non-negative matrix factorization method, a principal component analysis (PCA) method, a multi-dimensional scaling (MDS) method, a matrix decomposition based on eigenvalues method, a correspondence (CA) analysis method, a latent semantic analysis (LSA) method, a canonical correlation method, a factor analysis method, etc. These matrix factorization methods enable to provide a new expression (hereafter the projection matrix P1) in a new mathematical space (the projection space) of the initial matrix M1 on the basis of components that are at least partially decorrelated compared to the initial mathematical space of gene expression in which the initial matrix M1 is defined.

An example implementation using an independent component analysis (ICA) will be described in details hereafter.

After factorization, the matrix M1 may be expressed as

$$M1 \approx Q1 * P1 \qquad [Eq23]$$

where Q1 is a matrix (hereafter, the coefficient matrix) of size G*K1 and P1 is a matrix (hereafter, the projection matrix) of size K1*N1, where K1 is the number of components (hereafter, the ICA components) resulting from the factorization (e.g. the independent component analysis). Thus, the projection matrix P1 represents the projection of the N1 vectors on the K1 components resulting from the matrix factorization of the first matrix M1.

The stability and biological interpretation of the ICA components depend on the chosen number K1 of components. Each value of K1 will result in a different projection matrix P1 with K1 dimensions. Generally, the number K1 of components may be selected between 2 and 25 ($2 \leq K1 \leq 25$).

In step 230, among every K1 components of every projection matrices P1, the most discriminating component with respect to the tumor aggressiveness histological classes associated with the reference transcriptomic profiles is identified, thereby selecting the best P1 projection matrix into K1 components and $K1^M$ the most discriminating component.

In an alternate embodiment, the number K1 of components is predetermined (e.g. K1=2 or 3, on the basis of previous implementations of the steps 200-240 of a method for determining a reference molecular gradient) and during step 230, among the K1 components of the projection matrix P1, the most discriminating component with respect to the tumor aggressiveness histological classes associated with the reference transcriptomic profiles is identified, thereby selecting the $K1^M$ the most discriminating component.

In both embodiments, the purpose of step 230 is here to identify one component that allows to discriminate the transcriptomic profiles that belong to one tumor aggressiveness histological class with respect to other transcriptomic profiles that belong to another tumor aggressiveness histological class. This identification may be performed by evaluating a statistical association between the distribution of the projections of the N1 vectors on the K1 components and the tumor aggressiveness histological classes associated with the N1 vectors.

The evaluation of the statistical association may be performed using ANOVA (Analysis of Variance), linear models, generalized linear models, Kruskal-Wallis test, Pearson or Spearman correlation.

In step 240, the reference molecular gradient is computed as a distribution of projections of the N1 vectors on the $K1^M$ component. The distribution may be scaled to be defined on a range of easily interpretable values, for instance between 0 and 1 to be expressed as percentage or normalized around zero with zero mean and unit standard deviation.

To be able to determine which values of the distribution correspond to the higher (or respectively lower) histological grade, a correspondence between one or more selected tumor aggressiveness histological class(es) and a distribution of projections of vectors representing reference transcriptomic profiles belonging to the selected tumor aggressiveness histological class(es) is taken into account for determining the orientation of the projection value scale. Preferably, the tumor aggressiveness histological class representing the highest (or respectively lowest) aggressiveness is selected and the orientation of the projection value scale of the distribution is determined based on the correspondence between the selected histological class and a distribution of projections of vectors representing reference transcriptomic profiles belonging to the selected tumor aggressiveness histological class.

The scale of values may be normalized to facilitate interpretation of the projection value. For example, to get a normalized scale between [-1; +1], each projection value is normalized with respect to the mean value and / or lowest and highest values and / or standard deviation of the distribution.

The scaling function may be defined in different manner. For example, a normalized score $nsc$ may computed from a non-normalized value $sc$, the mean m and standard deviation $\sigma$ of the distribution corresponding to the reference molecular gradient. In an example, the standard deviation $\sigma$ is multiplied by a real number $w$ (scaling coefficient) that may be set between 1 and 3 ($1 \leq w \leq 3$). The scaling function is for example

$$nsc = \frac{sc - m}{w \cdot \sigma}$$

[Eq24]

The reference molecular gradient enables to represent a molecular grade associated with a transcriptomic profile using a single numerical value defined relatively to a scale of values.

The scale of values may be a defined by its upper and lower values such as [0, 100], [-100; +100], [0; 1] [-1, +1], etc. The tumor aggressiveness may be computed for any transcriptomic profile relatively to such a reference molecular gradient. This reference molecular gradient is also referred to therein as the reference tumor aggressiveness molecular gradient.

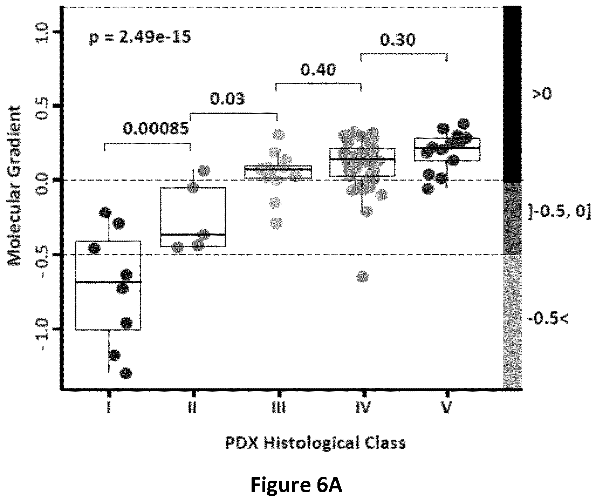

**Fig. 6a** that will be discussed in more details below show a diagram representing the molecular gradient as a function of the PDX histological class. This figure shows that:

- transcriptomic profiles belonging to the first histological class have a normalized score between approximately -1 and -0.2,
- transcriptomic profiles belonging to the second histological class have a normalized score between -0.5 and +0.1,
- transcriptomic profiles belonging to the third histological class have a normalized score between -0.3 and +0.3,
- transcriptomic profiles belonging to the fourth histological class have a normalized score between -0.2 and +0.3, and
- transcriptomic profiles belonging to the first histological class have a normalized score between -0.1 and +0.4;

Thus, based on this comparison, the higher values of the molecular gradient corresponds to higher values of aggressiveness and the orientation of the scale of the projection values is oriented accordingly: positive values of the scale [-1;+1] correspond to a high aggressiveness and negative values of the scale [-1;+1] correspond to a low aggressiveness.

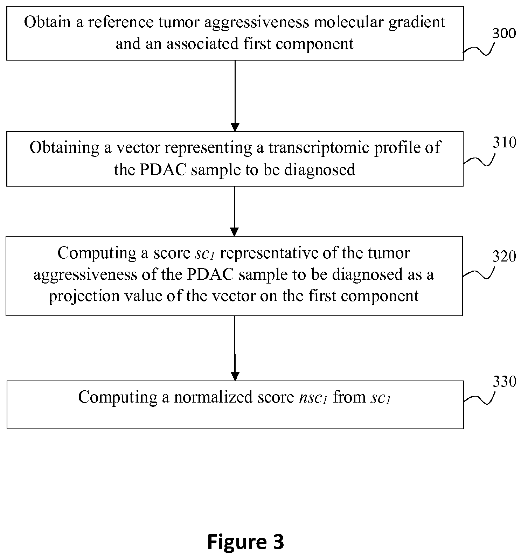

In one or more embodiments, the method steps 200-230 may be performed a first time with a first large set of genes until the coefficient matrix Q1 is obtained. Then only a reduced set of genes may be used. For example, the genes having the highest coefficients in absolute value in the coefficient matrix Q1 may be kept for the reduced set of genes. The method steps 200-240 may be performed again with this reduced set of genes to get a corresponding coefficient matrix Q1 and projection matrix P1 for the reduced set of genes. Thus, for the execution of the methods described below by reference to figures 3-5, only a reduced set of genes may be used, thereby simplifying the computations and limiting the resources that are necessary both for the RNA analysis and the computation of the matrices, projection values, etc.

For example, the gene expression is a RNA expression and the reduced set of genes includes part or all of the following genes: REG4, CTSE, CLDN18, TM4SF4, UGT2A3, SLC4A4, ANXA10, DPCR1, MUC17, HEPH, LYZ, TSPAN8 and TFF1.

Tumor aggressiveness based on a reference molecular gradient

A method for determining a tumor aggressiveness of a PDAC sample to be diagnosed on the basis of a reference molecular gradient will now be described by reference to **Fig. 3**.

While the steps are described in a sequential manner, the man skilled in the art will appreciate that some steps may be omitted, combined, performed in different order and / or in parallel.

In step 300, a reference molecular gradient is obtained. The reference molecular gradient may be obtained according to the method for determining a reference gradient disclosed by reference to **Fig. 2**. A first component $K1^M$ for which the reference molecular gradient is determined is thereby obtained.

In step 310 a vector $S1$ representing a transcriptomic profile of the PDAC sample to be diagnosed is obtained. The set of genes is the same as the set of genes used for determining the reference gradient. The transcriptomic profile of the PDAC sample is thus encoded by a vector $S1$ defined as:

$$S1 = \begin{bmatrix} y_1 \\ \vdots \\ y_G \end{bmatrix} \qquad\qquad [Eq31]$$

where G is the number of genes of the set of genes. The G genes used for determining the RNA expression of the PDAC sample to be diagnosed are the same as those used for generating the N1 reference vectors of the N1 reference transcriptomic profiles from which the reference molecular gradient was obtained in step 300.

Preferably, to enable a reliable comparison, the process for determining the transcriptomic profile of the PDAC sample to be diagnosed is the same as the process used for determining the N1 reference transcriptomic profiles from which the reference molecular gradient has been computed in step 300.

In step 320, a score $sc_1$ representative of the tumor aggressiveness of the PDAC sample is computed as the projection value of the vector $S1$ on the component $K1^M$ for which the reference tumor aggressiveness molecular gradient has been obtained.

The projection value may be obtained by inversing the coefficient matrix Q1, extracting the line vector $QV1$ corresponding to the component $K1^M$ and applying this line vector QV1 to the vector S1.

For example, the inverse matrix is defined as

$$QI^{-1} = \begin{bmatrix} q_{1,1} & \cdots & x1_{G,1} \\ \vdots & \ddots & \vdots \\ q_{1,K1} & \cdots & x1_{G,K1} \end{bmatrix}$$

$$[Eq32]$$

and the line vector $QV1$ corresponding to the $K1^M$ component may be defined as

$$QV1 = \begin{bmatrix} q_{1,K1^M} & \cdots & q_{G,K1^M} \end{bmatrix}$$

$$[Eq33]$$

thus

$$sc_1 = QV1 * S1 = \begin{bmatrix} q_{1,K1^M} & \cdots & q_{G,K1^M} \end{bmatrix} * \begin{bmatrix} y_1 \\ \vdots \\ y_G \end{bmatrix} = \sum_{j=1}^{G} q_{j,K1^M} * y_j$$

$$[Eq34]$$

In step 330, the score $sc_1$ is compared with the scale of the projection values of the distribution corresponding to reference tumor aggressiveness molecular gradient to determine a molecular grade. If a normalized scale is used in the reference tumor aggressiveness molecular gradient, then a normalized score $nsc_1$ is computed from the score $sc_1$ using the same scaling function as for the generation of the normalized scale (see for example above equation eq24).

Specific molecular gradient

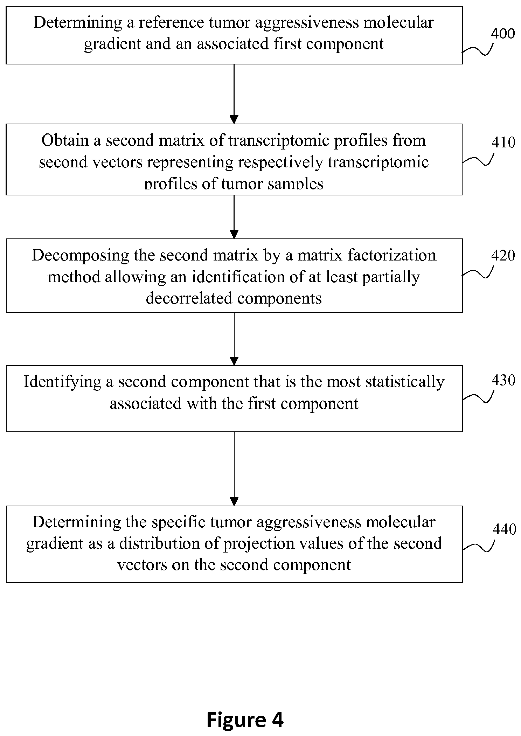

A method for determining a specific molecular gradient from a plurality of PDAC samples on the basis of a reference molecular gradient will now be described by reference to **Fig. 4**.

While the steps are described in a sequential manner, the man skilled in the art will appreciate that some steps may be omitted, combined, performed in different order and / or in parallel.

The method for determining a specific molecular gradient (also referred to as the specific tumor aggressiveness molecular gradient) may be performed only once a reference molecular gradient has been previously determined.

The specific molecular gradient is determined on the basis of second transcriptomic profiles generated for tumor samples. The second transcriptomic profiles (also referred to therein as the specific transcriptomic profiles) may be generated as explained in the present document or in any other manner. Especially, the process and / or technology used for generating these specific transcriptomic profiles may be different from the process and / or technology used for generating the reference transcriptomic profiles. To this extend, the specific molecular gradient is a molecular gradient that is specific to the process and / or technology used for generating these specific transcriptomic profiles and the specific molecular gradient may afterwards be used for determining tumor aggressiveness of a PDAC sample to be diagnosed based on an associated transcriptomic profile generated with the same process and / or technology.

In addition, in contrast with the determination of the reference molecular gradient, no tumor aggressiveness histological class is needed for these specific transcriptomic profiles since a comparison with the reference molecular gradient is used in replacement as will be explained below.

In a step 400, a reference molecular gradient is determined. The determination may be performed as described herein, for example by reference to **Fig. 2**. A first component $K1^M$ for which the reference molecular gradient is determined is thereby obtained.

In step 410, a second matrix M2 of transcriptomic profiles is generated from second vectors representing respectively the transcriptomic profiles of tumor samples. The samples may be of human or animal origin.

As already explained by reference to **Fig. 2**, a transcriptomic profile may be encoded as a data vector representing the gene expression, e.g. according to a RNA analysis of the tumor sample. The gene expression is determined with respect to a set of genes. Each vector coefficient of a data vector representing a transcriptomic profile is equal to the number of genes obtained for a given gene of the set of genes.

For the specific transcriptomic profiles, the RNA expression is based on a set of genes which is the same as the set of gene used for generating the reference molecular gradient. For example, the gene expression is a RNA expression and the set of genes includes part or all of the following genes: REG4, CTSE, CLDN18, TM4SF4, UGT2A3, SLC4A4, ANXA10, DPCR1, MUC17, HEPH, LYZ, TSPAN8 and TFF1.

Others sets of genes allow to validate the method of the present invention as defined in the annexed claims throughout numerous experiments carried out by the inventors. Without limitation of the scope of the invention, the inventors provide here some examples of other sets of genes allowing the generation of a reference molecular gradient as defined in the present invention:

- SPINK4, GP2, ITLN1, CASR, FABP1, DMBT1, ADRA2A, HMGCS2, BTNL8, SPINK1, LINC00675, MUC3A, IQGAP2, EDN3, CLCA1, TM4SF20, FER1L6, LGALS4, PHGR1, KCNE3, TM4SF5, NR0B2, FAM177B, CADPS, KCNJ3, SULT1B1, SULT1C2, ALDOB, ADH1C, ANXA13, IHH, PIP5K1B
- DPCR1, CLDN18, ADRA2A, SLC9A4, PGC, CTSE, PTGIS, VSIG1, ADH1C, CASR, REG4, ODAM, GCKR, NRG3, AKR1B10, NXF3, CYP2C9, AADAC, SERPINB2, CLDN2, NR0B2, FMOD, MUC17, SERPINB10, SSTR1, CAPN6, SULT1C2, PTGER2, PIGR, UGT2A3, ANXA10, GKN2, GCNT1, PSAPL1, NPC1L1, SCTR, MMP12, SUCNR1, KIAA1644, RNASE1, PRDM16, MUC6, CASP1, CD84, FGD5, KCNE3, A4GNT, CARD16, HEPH, IQGAP2, ONECUT3, GPRC5B, CADPS, GATA4, MXRA5, ADAMTS2, GKN1, KCNK10
- DPCR1, PTGER2, ADRA2A, CCL24, PIGR, DMBT1, HLA-DQB1, GABRP, CASP1, MUC17, SLC9A4, CFI, FGD5, ONECUT3, ANXA10, UST, PTPRZ1, ADAMTS2, EYA4, VSIG1, PARP15, ADH1C, CADPS, GATA4, GKN2, HTR2B, FAM153A, FAM131B, FGD2, GCNT1, ZNF300
- IGF2, H19, CACNG4, QPRT, INS-IGF2, GNG4, PEG10, PPP1R14A, ELF5, PRAME, LDHB, GMFG, HOXB13, TFAP2C, KCNMB4, SYT1, CYP24A1, FBXO17, PADI2, OLFML2A, SPOCK2, SGCE, MFAP2, APOE, ITPRIPL1, MERTK, IGDCC4
- DPCR1, PTGER2, ADRA2A, GABRP, PIGR, HLA-DQB1, MUC17, CCL24, CASP1, DMBT1, HLA-DRB1, GATA4, SLC9A4, ADH1C, HLA-DRA, ANXA10, FGD5, CFI, HLA-DRB5, VSIG1, PTPRZ1, NXF3, UST, FAM131B, FGD2, HTR2B, FMOD, EYA4, CAPN9, ONECUT3
- CLCA1, CDX1, SI, DMBT1, FABP2, MEP1A, SPINK4, ITLN1, CHGA, GP2, FABP1, NBPF7, PLA2G2A, HEPACAM2, CA8, RBP4, REG4, SLC6A7, ATOH1, SLC5A12, MUC17, KRT20, XPNPEP2, GALNT8, SLCO2A1, CLDN18, OLFM4, BTNL3, FMOD
- DPCR1, PTGER2, ADRA2A, PIGR, CCL24, DMBT1, GABRP, SLC9A4, HLA-DQB1, MUC17, CASP1, FGD5, VSIG1, ONECUT3, ANXA10, CADPS, UST, ADH1C, GATA4, PTPRZ1, GKN2, CFI, ADAMTS2, HTR2B, GCNT1, FMOD, EYA4, FGD2, FAM131B, DACH1, ALDH1A1, PIP5K1B, AADAC, ZNF300, GKN1, TLX1, BTNL9
- DPCR1, PGC, CLDN18, ADRA2A, SLC9A4, GKN2, GKN1, GCKR, NR0B2, PRDM16, PTGIS, MUC6, ODAM, PTGER2, SERPINB2, CASR, SERPINB10, SLC6A19, FMOD, NXF3, CTSE, SSTR1, ADH1C, MMP12, A4GNT, CADPS, VSIG1, PSAPL1, AKR1B10, AADAC, CAPN6, PRSS1, REG1A, MUC17, PLA2G2A

Let $N2$ be the number of second transcriptomic profiles and G be the number of genes in the set of genes used for the RNA expression. Then each transcriptomic profile $i$ ($1 \leq i \leq N2$) may be encoded by a second vector $V2_i$ of size G*1 defined as

$$V2i = \begin{bmatrix} x2_{i,1} \\ \vdots \\ x2_{i,G} \end{bmatrix} \qquad [Eq41]$$

where j is the gene subscript varying between 1 and G.

From the N2 vectors (hereafter the second vectors) representing the N2 reference transcriptomic profiles, a matrix M2 of vectors is generated. The size of the matrix M2 is G*N2 and the matrix M2 may be defined as

$$M2 = \begin{bmatrix} x2_{1,1} & \cdots & x2_{N2,1} \\ \vdots & \ddots & \vdots \\ x2_{1,G} & \cdots & x2_{N2,G} \end{bmatrix} \qquad [Eq42]$$

In step 420, the matrix M2 (hereafter the second matrix) is then factorized using a matrix factorization method allowing an identification of at least partially decorrelated components. Several matrix factorization methods may be used, for example an independent component analysis (ICA) method, a non-negative matrix factorization method, a principal component analysis (PCA) method, a multi-dimensional scaling (MDS) method, a matrix decomposition based on eigenvalues method, a correspondence (CA) analysis method, a latent semantic analysis (LSA) method, a canonical correlation method, a factor analysis method, etc.

An example implementation using an independent component analysis (ICA) will be described in details hereafter.

After factorization, the matrix M2 may be expressed as

$$M2 \approx Q2 * P2 \qquad [Eq43]$$

where Q2 is a matrix (hereafter the coefficient matrix) of size G2*K2 and P2 is a matrix (hereafter the projection matrix) of size K2*N2, where K2 is the number of components (hereafter ICA components) resulting from the factorization (e.g. the independent component analysis). Thus the projection matrix P2 represents the projection of the N2 vectors on the K2 components resulting from the matrix factorization.

The stability and biological interpretation of the ICA components depend on the chosen number K2 of components. Each value of K2 will result in a different projection matrix P2 with K2 dimensions. Generally, the number K2 of components may be selected between 2 and 25 ($2 \leq K2 \leq 25$).

In step 430, among every K2 components of every projection matrices P2, the most statistically associated with the first component $K1^M$ - for which the reference molecular gradient was previously obtained - is identified, thereby selecting the best P2 projections matrix into K2 components and $K2^M$ the component with the most statistical association with $K1^M$.

In an alternate embodiment, the number K2 of components is predetermined (e.g. K2=2 or 3, on the basis of previous implementations of the steps 400-440 of a method for determining a specific molecular gradient) and during step 430, among the K2 components of the projection matrix P2, the most discriminating component with respect to the tumor aggressiveness histological classes associated with the reference transcriptomic profiles is identified, thereby selecting the $K2^M$ the most discriminating component.

In both embodiments, the purpose of step 430 is to identify one component that allows to discriminate the transcriptomic profiles with respect to their tumor aggressiveness using the reference molecular gradient as reference in the absence of histological classes for the second transcriptomic profiles.

This identification of the component $K2^M$ that is the most statistically associated with the first component $K1^M$ may be performed by evaluating the statistical association between the distribution of the projections of the N2 second vectors on each of the K2 components and the distribution of the projections of the N2 vectors on the first component $K1^M$ (that was most to be the most discriminating during the determination of the reference molecular gradient).

Different statistical approaches may be used for the evaluation of the statistical association.

According to a first example, the identification of the second component $K2^M$ is performed by searching a correlation between the projections of the N2 second vectors on the K2 at least partially decorrelated components resulting from the matrix factorization of the second matrix M2 and the projections of the N2 second vectors on the first component $K1^M$. For this purpose, the N2 second vectors are projected using the line vector *QV1* of the inverse matrix *Q1⁻¹* and applying this line vector *QV1* to each concerned vector *V2i*:

$$q2_i = QV1 * V2_i = [q_{1,K1^M} \quad \cdots \quad q_{G,K1^M}] * \begin{bmatrix} x2_{i,1} \\ \vdots \\ x2_{i,G} \end{bmatrix} = \sum_{j=1}^{G} q_{j,K1^M} * x2_{i,j}$$

[Eq44]

Then the distribution of the values q2i is computed and compared with the distribution of the projection of the N2 second vectors on the each of the K2 components as given by the projection matrix P2.

According to a second example, the identification of the second component $K2^M$ is performed by searching a correlation between the K2 vectors of the coefficient matrix Q2 resulting from the matrix factorization of the second matrix M2 and the vector of the coefficient matrix Q1 resulting from the matrix factorization of the first matrix M1 corresponding to the first component $K1^M$.

In step 440, the reference molecular gradient is computed as a distribution of projections of the N2 vectors on the $K2^M$ component. The distribution may be scaled to be defined on a range of easily interpretable values, for instance between 0 and 1 to be expressed as percentage or normalized around zero with zero mean and unit standard deviation.

To be able to determine which values of the distribution correspond to the higher (or respectively lower) molecular grade, a correspondence between the reference tumor aggressiveness molecular gradient and the distribution of the projections of the second vectors on the second component is determined. In the case of an opposite correspondence between the first and second components, corresponding to a negative correlation, the values of the projection of the second component are transformed to obtain a positive correspondence, corresponding to a positive correlation.

Like the reference molecular gradient, this specific molecular gradient enables to represent a molecular grade associated with a transcriptomic profile using a single numerical value defined relatively to a scale of values (or range of values).

Like the reference molecular gradient, the scale of values may be defined by its upper and lower values such as [0, 100], [-100; +100], [0; 1] [-1, +1], etc. Thus, the tumor aggressiveness may be computed for any transcriptomic profile relatively to such a specific molecular gradient. This specific molecular gradient is also referred to herein as the specific tumor aggressiveness molecular gradient.

The scale of values may be normalized to facilitate interpretation of the projection value. For example, to get a normalized scale between [-1; +1], each projection value is normalized with respect to the mean value m and / or lowest and highest values and / or standard deviation of the distribution.

The scaling function may be defined in different manners. For example, a normalized score *nsc* may computed from a non-normalized value *sc*, the mean m and standard deviation $\sigma$ of the distribution corresponding to the reference molecular gradient. In an example, the standard deviation $\sigma$ is multiplied by a real number w (scaling coefficient) that may be set between 1 and 3 ($1 \le w \le 3$) . The scaling function is for example

$$nsc = \frac{sc - m}{w \cdot \sigma}$$

[Eq45]

Tumor aggressiveness based on a specific molecular gradient

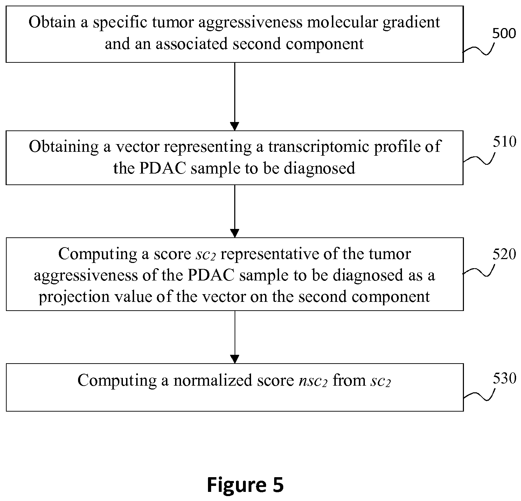

A method for determining tumor aggressiveness of a PDAC sample to be diagnosed on the basis of a specific tumor aggressiveness molecular gradient will now be described by reference to **Fig. 5**.

While the steps are described in a sequential manner, the man skilled in the art will appreciate that some steps may be omitted, combined, performed in different order and / or in parallel.

In step 500, a specific molecular gradient is obtained. The specific molecular gradient may be obtained according to the method for determining a specific molecular gradient disclosed by reference to Fig. 4. A second component $K2^M$ for which the specific molecular gradient is determined is thereby obtained.

In step 510 a vector S2 representing a transcriptomic profile of the PDAC sample to be diagnosed is obtained. The transcriptomic profile of the PDAC sample is encoded by a vector S2 defined as:

$$S2 = \begin{bmatrix} z_1 \\ \vdots \\ z_G \end{bmatrix} \qquad\qquad [Eq51]$$

where G is the number of gene of the set of genes. The G genes used for determining the RNA expression of the PDAC sample to be diagnosed are the same as those used for generating the N2 vectors of the N2 second transcriptomic profiles from which the specific molecular gradient was obtained.

Preferably, to enable a reliable comparison, the process for determining the transcriptomic profile of the PDAC sample to be diagnosed is the same as the process used for determining the N2 second transcriptomic profiles from which the specific molecular gradient has been computed in step 500.

In step 520, a score sc2 representative of the tumor aggressiveness of the PDAC sample is computed as the projection value of the vector S2 on the component K2$^M$ for which the specific molecular gradient has been obtained.

The projection value may be obtained by inversing the coefficient matrix Q2, extracting the line vector QV2 corresponding to the component K2$^M$ and applying this line vector QV2 to the vector S2.

For example, the inverse matrix is defined as

$$Q2^{-1} = \begin{bmatrix} p_{1,1} & \cdots & p_{G,1} \\ \vdots & \ddots & \vdots \\ p_{1,K1} & \cdots & p_{G,K2} \end{bmatrix}$$

[Eq52]

and the line vector *QV2* corresponding to the K2$^M$ component may be defined as

$$QV2 = \begin{bmatrix} p_{1,K2^M} & \cdots & p_{G,K2^M} \end{bmatrix}$$

[Eq53]

thus

$$SC_1 = QV2 * S2 = \begin{bmatrix} p_{1,K2^M} & \cdots & p_{G,K2^M} \end{bmatrix} * \begin{bmatrix} z_1 \\ \vdots \\ z_G \end{bmatrix} = \sum_{j=1}^{G} p_{j,K2^M} * z_j$$

[Eq54]

In step 530, the score $sc_2$ is compared with the scale of the projection values of the distribution corresponding to specific molecular gradient to determine a molecular grade representing the tumor aggressiveness.

If a normalized scale is used in the specific molecular gradient, then a normalized score $nsc_2$ is computed using the same scaling function as for the generation of the normalized scale (see for example above equation eq45).

It should be appreciated by those skilled in the art that any block diagrams herein represent conceptual views of illustrative circuitry embodying the principles of the invention. Similarly, it will be appreciated that any flow charts, flow diagrams, state transition diagrams, pseudo code, and the like represent various processes which may be substantially represented in computer readable medium and so executed by a computer or processor, whether or not such computer or processor is explicitly shown.

A further embodiment is a computer readable storage medium comprising computer program instructions for causing an apparatus to perform one or more steps of one or more of the methods described herein. The computer readable storage medium having computer readable program instructions embodied therein, which when being loaded on a computer, one or more processors and / or a programmable hardware component in an apparatus, cause the apparatus to perform one or more steps of one or more of the methods described herein. A further embodiment may be a computer program product comprising such a computer readable storage medium. The computer readable storage medium may be non-transitory.

A computer storage medium may be any physical media that can be read, written or more generally accessed by a computer. Embodiments of a computer-readable medium includes, but are not limited to, both computer storage media and communication media including any medium that facilitates transfer of a computer program from one place to another. Specifically, computer readable program instructions to perform embodiments described herein may be stored, temporarily or permanently, in whole or in part, on a non-transitory computer readable medium of a local or remote storage device including one or more storage media.

Examples of computer storage media include, but are not limited to, a flash drive or other flash memory devices (e.g. memory keys, memory sticks, USB key drive), CD-ROM or other optical storage, DVD, magnetic disk storage or other magnetic storage devices, solid state memory, memory chip, RAM, ROM, EEPROM, smart cards, a relational database management system, a traditional database, or any other suitable medium from that can be used to carry or store computer program instructions in the form of instructions or data structures which can be read by a computer processor. Also, various forms of computer-readable medium may be used to transmit or carry instructions to a computer, including a router, gateway, server, or other transmission device, wired (coaxial cable, fiber, twisted pair, DSL cable) or wireless (infrared, radio, cellular, microwave). The computer program instructions may include code from any computer-programming language, including, but not limited to, assembly, C, C++, Basic, SQL, MySQL, HTML, PHP, Python, R, Julia, Java, Javascript, etc.

A further embodiment of the invention is an apparatus comprising means configured to perform one or more steps of one or more methods described herein. The "means configured to perform" a given function shall be understood as functional block(s) comprising circuitry that is adapted for performing or configured to perform the given function. Moreover, any entity described herein as "means", may correspond to or be implemented as "one or more modules", "one or more devices", "one or more units", etc. Means for performing one or more functions may also comprises one or more processors and one or more memories (e.g. in a system or apparatus) for storing computer program instructions configured to, when executed by at least one processor, cause the performance (e.g. by the system or apparatus) of the one or more functions.

When provided by a processor, the functions may be provided by a single dedicated processor, by a single shared processor, or by a plurality of individual processors, some of which may be shared. Moreover, explicit use of the term "processor" may implicitly include, digital signal processor (DSP) hardware, network processor, application specific integrated circuit (ASIC), field programmable gate array (FPGA), etc. Other hardware, conventional or custom, may also be included. Their function may be carried out through the operation of program logic, through dedicated logic, through the interaction of program control and dedicated logic, or even manually, the particular technique being selectable by the implementer as more specifically understood from the context.

A memory may include a random access memory (RAM), cache memory, non-volatile memory, backup memory (e.g., programmable or flash memories), read-only memory (ROM), a hard disk drive (HDD), a solid state drive (SSD) or any combination thereof. The ROM may be configured to store, amongst other things, an operating system of the system / apparatus and / or computer program instructions. The RAM may be used by the processor(s) for the temporary storage of data.

Survival analysis

To assess the relationship between the molecular gradient and Overall Survival from diagnosis, we performed regression analysis with cox proportional hazard model (Gwen Lomberk et al. 2018 **[13]**). The Kaplan-Meier and risk tables was generate from the package survminer.(Jiri Lenz et al. 2011 **[14]**). The forest plots were generated from the package forest model (P. Vilmann et al, 1992 **[15]**)

Unravelling a molecular gradient

Although the histological characterization of PDX is a valuable prognostic tool, it is not compatible with clinical routine. In order to make this curated PDX histological classification assessable in clinically compatible samples, we investigated whether genome-wide molecular profiles could be applied to transfer our findings. We used an unsupervised statistical approach (using for example an ICA, Independent Component Analysis) to extract molecular gradient from the transcriptomic profiles.

Results of the statistical analysis

**Figs. 6a-6c** show the association between the molecular gradient and the PDX histological classes, in which:

- **Fig. 6a**. shows box and whiskers plot of the 5 PDX histopathology classes characterized by the molecular gradient estimated by the transcriptomes of the PDX tumors;

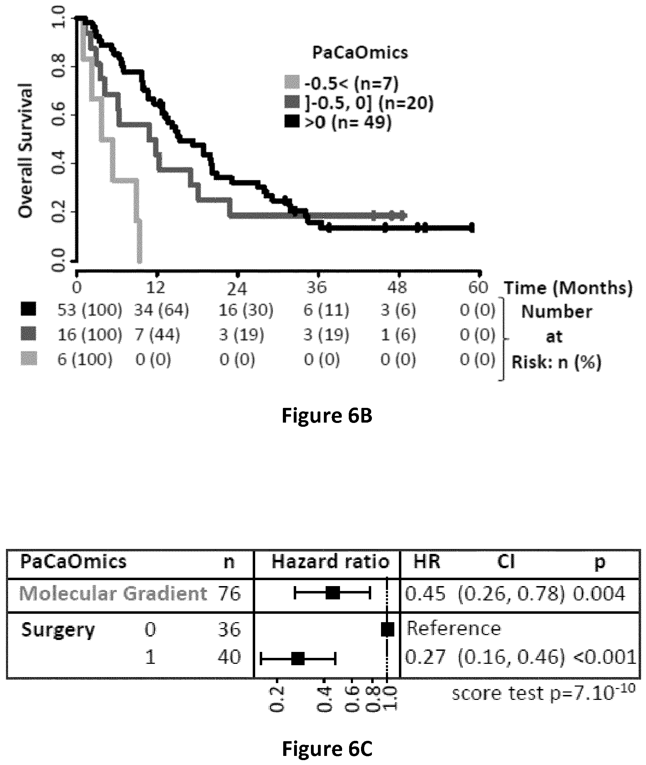

- **Fig. 6b** shows forest plot of the multivariate OS analysis for the PDX molecular gradient with the patient's surgical status (0 = no curative surgery versus 1 = curative surgery). Hazard Ratio (HR) and 95% Confidence Interval (CI);
- **Fig. 6c** shows OS curves of the 76 PDAC PDX from the PaCaOmics cohort. Patients are stratified by three arbitrary cut-offs of the molecular gradient as measured by the transcriptome of their corresponding PDX.

We identified an RNA-based component that is highly associated with the five PDX histological classes I-V, and therefore used the RNA-based component as for the determination of the reference molecular gradient (**Figs. 6a-6c**). This reference molecular gradient is used as a molecular grading system and represent a distribution of scores computed from a weighted combination of gene expression values. The distribution may be standardized around zero with non-outlier values between -1 and +1.

In addition to being the molecular counterpart of the histological classes (**Fig. 6a**), this molecular gradient was also associated with patient OS both alone (univariate Hazar Ratio (HR)= 0.35 ; 95% Confidence Interval (CI) [0.21-0.59] ; p-value <0.001) or independently of curative surgery (multivariate HR= 0.45 ; 95% CI [0.26-0.78] ; p-value= 0.004) (**Fig. 6b**). The aim of the molecular grading system disclosed herein is to define a continuous scale, for which each sample is given a numerical value, thereby placing it in a gradient of PDAC aggressiveness.

For illustrative purposes, thresholds corresponding to the differences between class I, II and III (i.e. -½ and 0) were used to subdivide the gradient into three, providing groups for Kaplan-Meier survival curves as shown in Fig. 6c.

Molecular grading of human primary PDAC

In order to transfer the molecular grading system from PDX tumor transcriptomic profiles to clinically compatible samples, the generation of a reference molecular gradient described herein enables to extract the matching molecular grade in an independent transcriptomic dataset. The hereby described statistical approach is technology-agnostic as it can be applied to any genome-wide RNA quantification profiles.

Moreover, the transcriptomic decomposition allows the identification of the molecular gradient independently of the presence of non-neoplastic tissue cells, provided it is detectable in the remaining tumor cells.

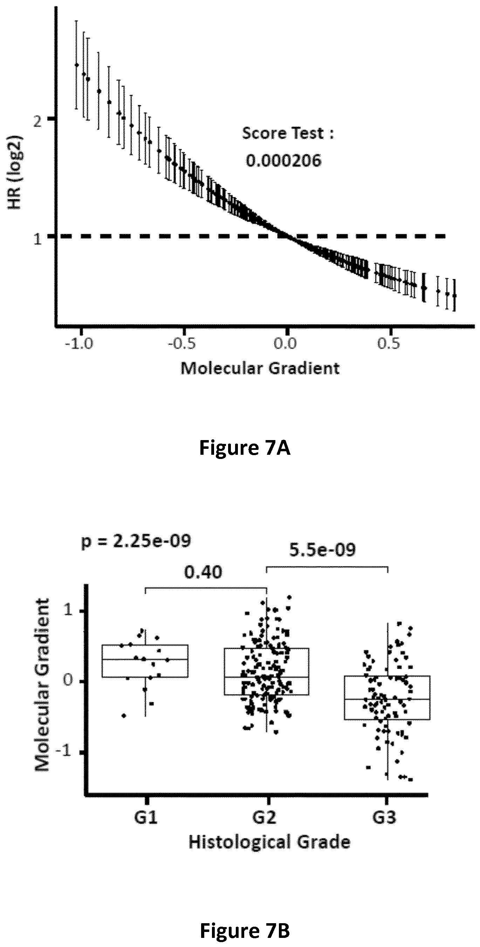

In order to evaluate the molecular gradient in human primary PDAC, its reproducibility and association with survival was assessed in public datasets of PDAC transcriptomic profiles. Remarkably, the molecular grade was unambiguously identified in all the tested PDAC transcriptomic datasets. We first evaluated the molecular grade in two datasets of 269 and 92 resected PDAC samples, analysed by microarray or RNA-seq respectively, from the Australian ICGC project (Bailey et al. [12]). Prognostic values of the molecular grading system are illustrated by **Figs. 7a-7d**, **8a-8d** and **9a-9d** respectively and will be presented in details below.

**Figs. 7a-7d** show prognostic value of the molecular grading system in ICGC series of resected PDAC analysed by microarray, in which:

- **Fig. 7a** shows patients OS relative risk estimated with the cox proportional hazard regression model illustrating the association between survival and the transcriptome-based estimation of the molecular gradient (n=258);
- **Fig. 7b** shows Kaplan-Meier OS curves stratified by cut-offs of the molecular gradient;

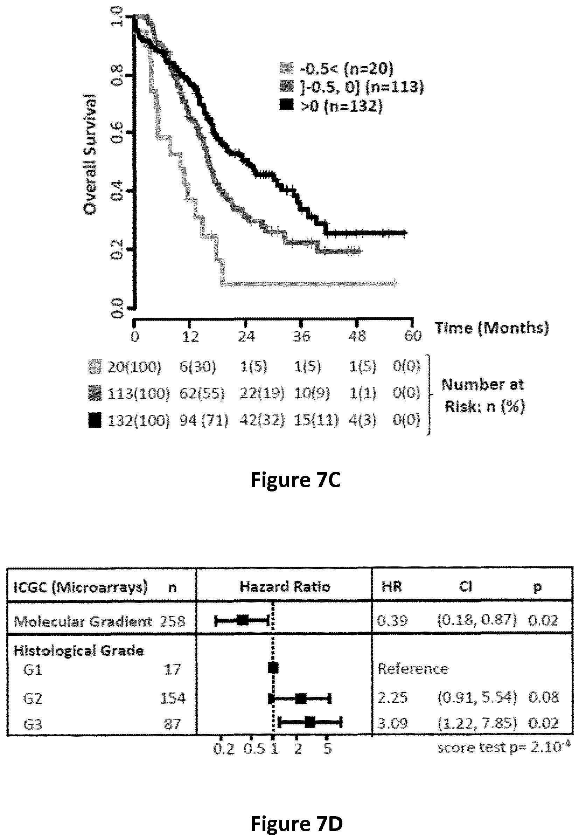

- **Fig. 7c** shows a box and whisker plot of the association between the molecular gradient and the histological grade. Reported p-values are from ANOVA and pairwise t-tests with holms correction;
- **Fig. 7d** shows a forest plot of the multivariate survival cox proportional hazard model for the molecular gradient and histological grade. Hazard Ratio (HR) and 95% confidence interval (CI) are shown.

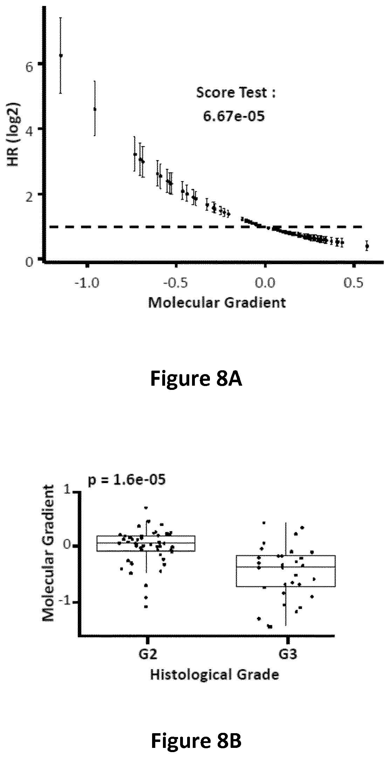

**Figs. 8a-8d** show prognostic value of the molecular grading system in ICGC series of resected PDAC analysed by RNA-seq technology, in which:

- **Fig. 8a** shows patients OS relative risk estimated with the cox proportional hazard regression model illustrating the association between survival and the transcriptome-based estimation of the molecular gradient (n=92);
- **Fig. 8b** shows Kaplan-Meier OS curves stratified by cut-offs of the molecular gradient;

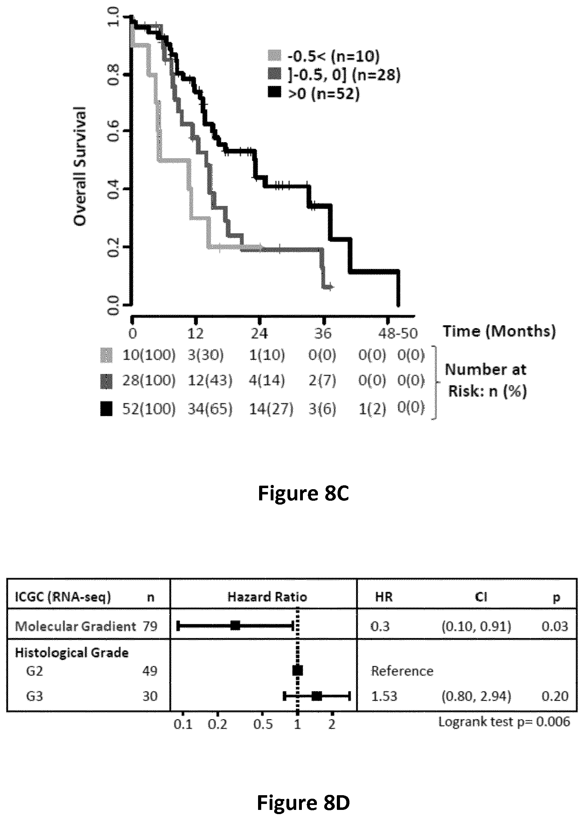

- **Fig. 8c** shows a box and whisker plot of the association of the molecular gradient and histological grade. Reported p-value corresponds to Student's t-test;
- **Fig. 8d** Forest plot of the multivariate survival cox proportional hazard model for the molecular gradient and histological grade. The plot shows the Hazard Ratio (HR) with the 95% CI;

Results

In the microarray dataset, the molecular grade was highly associated with the OS of patients, as shown by an increased relative risk of decease for patients with more aggressive molecular gradient, i.e. lower molecular gradient score (HR= 0.26, 95% CI [0.13-0.55], p-value <0.001) (**Figs. 7a** and **7b**). Median OS ranged from 10.1 to 35.8 months for respectively the bottom and top 10% of patients along the molecular gradient. The histopathological grading of these resected tumors was associated with their molecular grade (**Fig. 7c**). In a multivariate cox proportional hazard model including both the histopathology grading and the molecular grade, the molecular grade remained an independent predictor of OS (HR= 0.39, 95% CI [0.18-0.84], p-value=0.02; **Fig. 7d**).

Remarkably, similar results were found in the RNA-seq dataset consisting of 92 resected PDAC as presented in **Fig. 8a-8d**.

Discussion

The advent of high-throughput sequencing technologies has provided the opportunity to more conveniently and affordably portray tumor whole-transcriptomes in clinical settings.(Pauline Duconseil et al 2015 **[16]**; Bullard et al 2010 **[23]**). These technologies have resulted in the identification of numerous gene signatures in PDAC (Moffitt RA 2015 [24]; Maurer HC et al, 2019 [25]). In fact, high-throughput sequencing provides the necessary tools to identify, with unprecedented power and precision, underlying phenotypes of tumors that would otherwise be missed by histology. Defining clinically relevant phenotypes in PDAC could lead to improvements in overall outcomes by facilitating clinical management. Currently, therapeutic decisions in PDAC are made regardless of tumor grade and based only on clinico-radiological criteria. Being able to determine the prognosis more precisely by defining the grade of aggressiveness at the time of diagnosis would improve patient care. For instance, tumor resection could be considered in patients with large tumors invading the mesenteric vessels if its phenotype associated grade predicts good prognosis.3 Conversely, a small tumor without contraindication to surgery but with a grading predicting a poor prognosis could be not immediately operated but treated by chemotherapy before surgery.

PDAC tumor aggressiveness is currently only determined for the small subset of operated patients by describing the visible state of differentiation of tumor cells. In this study, we propose to measure the differentiation phenotype of a tumor through its RNA profile. We defined an original bioinformatics strategy based on transcriptomics that is able to identify the level of aggressiveness of a PDAC tumor sample. The proposed molecular grading system expresses the continuous grade of differentiation of tumors based on their transcriptomic profile. This strategy resulted in a transcriptomic reference scale ranging from poorly- to well-differentiate and in which each new sample can be matched, allowing the classification of PDAC into a clinically relevant scale. Interestingly, the efficiency of this approach is independent of the method used to obtain the transcriptome data since RNA-seq or microarray gave remarkable results, indicating the robustness of the proposed algorithm. Another important finding is that this strategy can be applied not only to resected patients, for which the biological material is abundant and the RNA-based molecular grading matches the histological analysis, but also on biopsy samples in which assessing the level of differentiation can be difficult.

Identified from a prospective cohort of both resectable and unresectable PDAC, the molecular grade efficiently distinguishes patients with a longer life expectancy from those with a rapid progressive disease. In the particular case of borderline patients, were resectability is difficult to be determined, molecular grading is highly valuable to help surgery management. Indeed, patients that present local invasion or resectable metastasis could highly benefit from surgery if molecular grading predicts a better prognostic.

In conclusion, disclosed is a universal molecular tool that can be used on any PDAC sample to grade the tumor's aggressivity. This molecular gradient may be used, in combination with current anatomopathological characteristics when possible.

**List of References**

[1] Deramaudt T, Rustgi AK, Mutant KRAS in the initiation of pancreatic cancer, Biochim Biophys Acta. 2005 Nov 25; 1756(2):97-101.

[2] Feldmann et al., Molecular genetics of pancreatic intraepithelial neoplasia, J Hepatobiliary Pancreat Surg. 2007; 14(3):224-32. Epub 2007 May 29.

[3] Iovanna et al., Current knowledge on pancreatic cancer. Front Oncol. 2012; 2:6. doi: 10.3389/fonc.2012.00006. eCollection 2012.

[4] Ying et al., Genetics and biology of pancreatic ductal adenocarcinoma. Genes Dev. 2016; 30(4):355-85. doi: 10.1101/gad.275776.115.

[5] Ryan Carr and Martin E. Fernandez-Zapico, Pancreatic cancer microenvironment, to target or not to target?, EMBO Mol Med. 2016 Feb; 8(2): 80-82.

[6] Venu G Pillarisetty, The pancreatic cancer microenvironment: an immunologic battleground, OncoImmunology 3:8, e950171; August 1, 2014.

[7] J. Iovanna and Closa, "Factors released by the tumor far microenvironment are decisive for pancreatic adenocarcinoma development and progression", Oncoimmunology, 2017 Aug 8;6(11).

[8] Leca, J. et al. Cancer-associated fibroblast-derived annexin A6+ extracellular vesicles support pancreatic cancer aggressiveness. J Clin Invest. 2016; 126(11):4140-4156. doi: 10.1172/JCI87734. Epub 2016 Oct 4.

[9] HA Burris et al., Improvements in survival and clinical benefit with gemcitabine as first-line therapy for patients with advanced pancreas cancer: a randomized trial, J Clin Oncol. 1997 Jun;15(6):2403-13.

[10] Thierry Conroy et al., FOLFIRINOX versus Gemcitabine for Metastatic Pancreatic Cancer, New England Journal of Medicine, 2011; 364:1817-1825 / DOI: 10.1056/NEJMoa1011923.

[11] Nicolle et al., Pancreatic Adenocarcinoma Therapeutic Targets Revealed by Tumor-Stroma Cross-Talk Analyses in Patient-Derived Xenografts, Cell Rep. 2017 Nov 28;21(9):2458-2470.

[12] Bailey P et al. Australian Pancreatic Cancer Genome Initiative. Genomic analyses identify molecular subtypes of pancreatic cancer; Nature 2016;531:47-52.

[13] Gwen Lomberk et al., Distinct epigenetic landscapes underlie the pathobiology of pancreatic cancer subtypes,, NATURE COMMUNICATIONS (2018) 9:1978, DOI: 10.1038/s41467-018-04383-6 / www.nature.com/naturecommunications.

[14] Jiri Lenz et al., Clinicopathological correlations of nestin expression in surgically resectable pancreatic cancer including an analysis of perineural invasion; Journal of gastrointestinal and liver diseases: JGLD. 20(4), 389-396 (2011).

[15] P. Vilmann et al., Endoscopic ultrasonography with guided fine needle aspiration biopsy in pancreatic disease, Gastrointest Endosc. 1992 Mar-Apr; 38(2):172-3.

[16] Pauline Duconseil et al., Transcriptomic Analysis Predicts Survival and Sensitivity to Anticancer Drugs of Patients with a Pancreatic Adenocarcinoma, The American Journal of Pathology, Vol. 185, No. 4, April 2015.

[17] Kulkarni MM. Digital multiplexed gene expression analysis using the NanoString nCounter system. Curr Protoc Mol Biol. 2011 Apr;Chapter 25:Unit25B.10. doi:10.1002/0471142727.mb25b10s94. PubMed PMID: 21472696.

[18] Marioni JC, Mason CE, Mane SM, Stephens M, Gilad Y. RNA-seq: an assessment of technical reproducibility and comparison with gene expression arrays. Genome Res. 2008 Sep;18(9):1509-17. doi: 10.1101/gr.079558.108. Epub 2008 Jun 11. PubMedPMID: 18550803; PubMed Central PMCID: PMC2527709.

[19] Wang Z, Gerstein M, Snyder M. RNA-Seq: a revolutionary tool for transcriptomics. Nat Rev Genet. 2009 Jan;10(1):57-63. doi: 10.1038/nrg2484. Review. PubMed PMID: 19015660; PubMed Central PMCID: PMC2949280.

[20] Steinbock LJ, Radenovic A. The emergence of nanopores in next-generation sequencing. Nanotechnology. 2015; 26(7):074003. doi: 10.1088/0957-4484/26/7/074003. Epub 2015 Feb 2.

[21] Dobin A, Davis CA, Schlesinger F, Drenkow J, Zaleski C, Jha S, Batut P, Chaisson M, Gingeras TR. STAR: ultrafast universal RNA-seq aligner, Bioinformatics, 2013.

[22] Jan 1;29(1):15-21. doi: 10.1093/bioinformatics/bts635. Epub 2012 Oct 25. PubMed PMID: 23104886; PubMed Central PMCID: PMC3530905. Liao Y, Smyth GK and Shi W. featureCounts: an efficient general-purpose program for assigning sequence reads to genomic features. Bioinformatics, 30(7):923-30, 2014

[23] Bullard et al., Evaluation of statistical methods for normalization and differential expression in mRNA-Seq experiments, BMC Bioinformatics 2010 11:94 / http://www.biomedcentral.com/1471-2105/11/94.

[24] Moffitt RA, Marayati R, Flate EL, et al: Virtual microdissection identifies distinct tumor- and stroma-specific subtypes of pancreatic ductal adenocarcinoma. Nat Genet 47:1168-1178, 2015

[25] Maurer HC, Holmstrom SR, He J, et al: Experimental microdissection enables functional harmonisation of pancreatic cancer subtypes. Gut , 2019

**Claims**

1. A method for determining a reference tumor aggressiveness molecular gradient for at least one pancreatic ductal adenocarcinoma (PDAC) sample, the method comprising:

   - generating a first matrix of transcriptomic profiles from first vectors representing respectively reference transcriptomic profiles of tumor samples of human or animal origin;
   - obtaining, for each reference transcriptomic profile, a tumor aggressiveness histological class;
   - decomposing the first matrix by a matrix factorization method allowing an identification of at least partially decorrelated components;
   - identifying a first component that is the most discriminating relatively to the tumor aggressiveness histological classes associated with the reference transcriptomic profiles;
   - determining the reference tumor aggressiveness molecular gradient as a distribution of projection values of the first vectors on the first component, wherein the orientation of the scale of the projection values is determined on the basis of the correspondence between at least one tumor aggressiveness histological class and the distribution of the projection values of the first vectors representing reference transcriptomic profiles belonging to said at least one tumor aggressiveness histological class.

2. The method according to claim 1, wherein the most discriminating component is identified by evaluating a statistical association between the distribution of the projections of the first vectors on the at least partially decorrelated components and the tumor aggressiveness histological classes.

3. The method according to claim 2, wherein the statistical association is performed using an algorithm based on an Analysis of Variance, ANOVA, linear models, generalized linear models, a Kruskal-Wallis test, or a Pearson or Spearman correlation test.

4. A method for determining a tumor aggressiveness of a PDAC sample to be diagnosed, the method comprising:

   - obtaining a vector representing a transcriptomic profile of the PDAC sample to be diagnosed;
   - obtaining a reference tumor aggressiveness molecular gradient by the method according to any of claims 1 to 3;
   - computing a score representative of the tumor aggressiveness of the PDAC sample as the projection value of the vector representing the transcriptomic profile of the PDAC sample on the first component for which the reference tumor aggressiveness molecular gradient has been obtained; and
   - comparing the score with the reference tumor aggressiveness molecular gradient.

5. The method according to claim 4 further comprising :

   - computing a normalized score from the score and the mean and standard deviation of the reference tumor aggressiveness molecular gradient.

6. A method for determining a specific tumor aggressiveness molecular gradient from a plurality of PDAC samples, the method comprising:

   - generating a second matrix of transcriptomic profiles from second vectors representing respectively specific transcriptomic profiles of the plurality of PDAC samples;
   - decomposing the second matrix by a matrix factorization method allowing an identification of at least partially decorrelated components;
   - determining a first component and a reference tumor aggressiveness molecular gradient by the method according to any of claims 1 to 3;
   - identifying a second component that is the most statistically associated with the first component for which the reference tumor aggressiveness molecular gradient has been obtained;
   - determining the specific tumor aggressiveness molecular gradient as a distribution of projection values of the second vectors on the second component, wherein the orientation of the scale of the projection values is determined on the basis of the correspondence between the reference tumor aggressiveness molecular gradient and the distribution of the projection values of the second vectors on the second component.

7. The method according to claim 6, wherein the identification of the second component is performed by searching a correlation between the projections of the second vectors on the at least partially decorrelated components resulting from the matrix factorization of the second matrix and the projections of the second vectors on the first component.

8. The method according to claim 6, wherein the identification of the second component is performed by searching a correlation between the vectors of the coefficient matrix resulting from the matrix factorization of the second matrix and the vector of the coefficient matrix resulting from the matrix factorization of the first matrix corresponding to the first component.

9. A method for determining tumor aggressiveness of a PDAC sample to be diagnosed, the method comprising:

   - obtaining a vector representing a transcriptomic profile of the PDAC sample to be diagnosed;
   - obtaining a second component and a specific tumor aggressiveness molecular gradient by a method according to any of claims 6 to 8;
   - computing a score representative of the tumor aggressiveness of the PDAC sample as the projection value of the vector representing the transcriptomic profile of the PDAC sample on the second component for which the specific tumor aggressiveness molecular gradient has been obtained; and
   - comparing the score with the specific tumor aggressiveness molecular gradient.

10. The method according to claim 9, further comprising :

   - computing a normalized score from the score and the mean and standard deviation of the specific tumor aggressiveness molecular gradient.

11. The method according to any of claims 1 to 10, wherein the matrix factorization method is selected among independent component analysis method, a non-negative matrix factorization method, a principal component analysis method, a multi-dimensional scaling method, a decomposition based on eigenvalues method, a correspondence analysis method, a latent semantic analysis method, a canonical correlation method, a factor analysis method.

12. The method according to any of claims 1 to 10, wherein the matrix factorization method is an independent component analysis and the first component is the first independent component that is the most discriminating relatively to the tumor aggressiveness histological classes associated with the reference transcriptomic profiles.

13. The method according to any of claims 1 to 12, wherein the reference transcriptomic profiles of tumor samples of human origin are obtained from patients-derived tumor xenografts.

14. The method according to claim 13, wherein the tumor aggressiveness histological classes are obtained from said patients-derived tumor xenografts.

15. A computer readable medium comprising computer program instructions which when executed causes an apparatus to perform the steps of the method according to any of claims 1 to 14.

16. An apparatus comprising means for performing the steps of the method according to any of claims 1 to 14.

| Representative PDX | | | | | |
|---|---|---|---|---|---|
| Class | I | II | III | IV | V |
| Differentiation | Poor | | Moderately | well | |
| Proportion | 10.53% | 6.58% | 15.79% | 50.00% | 17.11% |

**Figure 1a**

**Figure 1b**

Chi-squared test: p-value < 0.05

**Figure 1c**

| Obtain a matrix of transcriptomic profiles from first vectors representing respectively reference transcriptomic profiles of tumor samples | 200 |

| Obtaining, for each reference transcriptomic profile, a tumor aggressiveness histological class | 210 |

| Decomposing the matrix by a matrix factorization method allowing an identification of at least partially decorrelated components | 220 |

| Identifying a first component that is the most discriminating relatively to the tumor aggressiveness histological classes associated with the reference transcriptomic profiles | 230 |

| Determining the reference tumor aggressiveness molecular gradient as a distribution of projection values of the first vectors on the first component | 240 |

**Figure 2**

Obtain a reference tumor aggressiveness molecular gradient and an associated first component — 300

Obtaining a vector representing a transcriptomic profile of the PDAC sample to be diagnosed — 310

Computing a score $sc_1$ representative of the tumor aggressiveness of the PDAC sample to be diagnosed as a projection value of the vector on the first component — 320

Computing a normalized score $nsc_1$ from $sc_1$ — 330

**Figure 3**

Determining a reference tumor aggressiveness molecular gradient and an associated first component ⟶ 400

Obtain a second matrix of transcriptomic profiles from second vectors representing respectively transcriptomic profiles of tumor samples ⟶ 410

Decomposing the second matrix by a matrix factorization method allowing an identification of at least partially decorrelated components ⟶ 420

Identifying a second component that is the most statistically associated with the first component ⟶ 430

Determining the specific tumor aggressiveness molecular gradient as a distribution of projection values of the second vectors on the second component ⟶ 440

**Figure 4**

| Obtain a specific tumor aggressiveness molecular gradient and an associated second component | 500 |

↓

| Obtaining a vector representing a transcriptomic profile of the PDAC sample to be diagnosed | 510 |

↓

| Computing a score $sc_2$ representative of the tumor aggressiveness of the PDAC sample to be diagnosed as a projection value of the vector on the second component | 520 |

↓

| Computing a normalized score $nsc_2$ from $sc_2$ | 530 |

**Figure 5**

**Figure 6A**

**Figure 6B**

**Figure 6C**

**Figure 7A**

**Figure 7B**

**Figure 7C**

| ICGC (Microarrays) | n | Hazard Ratio | HR | CI | p |
|---|---|---|---|---|---|
| Molecular Gradient | 258 | | 0.39 | (0.18, 0.87) | 0.02 |
| **Histological Grade** | | | | | |
| G1 | 17 | | Reference | | |
| G2 | 154 | | 2.25 | (0.91, 5.54) | 0.08 |
| G3 | 87 | | 3.09 | (1.22, 7.85) | 0.02 |

0.2 0.5 1 2 5      score test p= $2.10^{-4}$

**Figure 7D**

**Figure 8A**

**Figure 8B**

**Figure 8C**

**Figure 8D**

Europäisches
Patentamt

European
Patent Office

Office européen
des brevets

**EUROPEAN SEARCH REPORT**

Application Number

EP 19 30 5522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/061252 A1 (UNIV NORTH CAROLINA [US]) 21 April 2016 (2016-04-21) * the whole document * * page 2, line 26 - page 6, line 10; claims 1-14; figures 1-21 * * page 3, line 11 - page 11, line 20 * * page 15, line 2 - page 16, line 2 * * page 56, line 5 - page 76; examples 1-9 * | 1-16 | INV. G01N33/574 G16B20/00 |
| Y | LAN ZHAO ET AL: "Gene expression profiling of 1200 pancreatic ductal adenocarcinoma reveals novel subtypes", BMC CANCER, vol. 18, no. 1, 29 May 2018 (2018-05-29), XP055629250, DOI: 10.1186/s12885-018-4546-8 * the whole document * * abstract * * figures 1-5; tables 1, 2 * | 1-16 | |
| Y | WO 2018/095933 A1 (UNIV DAIX MARSEILLE AMU [FR]; CENTRE NAT RECH SCIENT [FR] ET AL.) 31 May 2018 (2018-05-31) * the whole document * * pages 11-12; tables 1,2 * * page 22, line 5 - page 25, line 22 * * page 31, line 20 - page 35, line 30; claims 1-15; figures 1-7 * | 1-16 | TECHNICAL FIELDS SEARCHED (IPC) G01N G06F C12Q G16B |
| Y | US 2017/277826 A1 (OZEROV IVAN [US] ET AL) 28 September 2017 (2017-09-28) * the whole document * * paragraphs [0014] - [0058]; claims 1-17; figures 1-6 * | 1-16 | |

-/--

The present search report has been drawn up for all claims

| Place of search | Date of completion of the search | Examiner |
|---|---|---|
| The Hague | 17 October 2019 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

Application Number

EP 19 30 5522

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2013/040251 A2 (ASURGEN INC [US]; ADAI ALEX [US] ET AL.) 21 March 2013 (2013-03-21) * the whole document * * paragraphs [0011] - [0089]; claims 1-206; figures 1-18 * ----- | 1-16 | |
| Y | WO 2014/056626 A1 (HI STEM GGMBH [DE]) 17 April 2014 (2014-04-17) * the whole document * ----- | 1-16 | |
| Y | WO 2015/171736 A2 (UNIV UTAH RES FOUND [US]) 12 November 2015 (2015-11-12) * the whole document * * paragraphs [0006] - [0025], [0078], [0133]; figures 1-10A * ----- | 1-16 | |
| Y | WO 02/103320 A2 (ROSETTA INPHARMATICS INC [US]) 27 December 2002 (2002-12-27) * the whole document * * figure 2 * * figures 1-32 * ----- | 1-16 | TECHNICAL FIELDS SEARCHED (IPC) |
| Y | US 2015/018230 A1 (KORC MURRAY [US] ET AL) 15 January 2015 (2015-01-15) * the whole document * ----- | 1-16 | |
| Y | WO 2016/045799 A1 (HI STEM GGMBH [DE]) 31 March 2016 (2016-03-31) * the whole document * * paragraphs [0115] - [0120]; examples 1-4 * ----- | 1-16 | |
| Y | WO 2014/056627 A1 (HI STEM GGMBH [DE]) 17 April 2014 (2014-04-17) * the whole document * ----- | 1-16 | |

-/--

The present search report has been drawn up for all claims

| Place of search | Date of completion of the search | Examiner |
|---|---|---|
| The Hague | 17 October 2019 | Boiangiu, Clara |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 19 30 5522

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/120069 A1 (BETH ISRAEL HOSPITAL [US]) 13 August 2015 (2015-08-13) * the whole document * | 1-16 | |
| Y | WO 2017/180886 A1 (MAYO FOUNDATION [US]) 19 October 2017 (2017-10-19) * the whole document * | 1-16 | |
| Y | WO 2017/079632 A1 (CEDARS-SINAI MEDICAL CENTER [US]) 11 May 2017 (2017-05-11) * the whole document * | 1-16 | |
| Y | WO 2013/060739 A1 (CHUNDSELL MEDICALS AB [SE]) 2 May 2013 (2013-05-02) * the whole document * | 1-16 | |
| Y | STEIN-O'BRIEN GENEVIEVE L ET AL: "Enter the Matrix: Factorization Uncovers Knowledge from Omics", TRENDS IN GENETICS, ELSEVIER SCIENCE PUBLISHERS B.V. AMSTERDAM, NL, vol. 34, no. 10, 22 August 2018 (2018-08-22), pages 790-805, XP085478880, ISSN: 0168-9525, DOI: 10.1016/J.TIG.2018.07.003 * the whole document * | 1-16 | **TECHNICAL FIELDS SEARCHED (IPC)** |

The present search report has been drawn up for all claims

| Place of search | Date of completion of the search | Examiner |
|---|---|---|
| The Hague | 17 October 2019 | Boiangiu, Clara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 30 5522

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2019

| Patent document cited in search report | | Publication date | Patent family member(s) | | Publication date |
|---|---|---|---|---|---|
| WO 2016061252 | A1 | 21-04-2016 | US | 2017233827 A1 | 17-08-2017 |
| | | | WO | 2016061252 A1 | 21-04-2016 |
| WO 2018095933 | A1 | 31-05-2018 | NONE | | |
| US 2017277826 | A1 | 28-09-2017 | NONE | | |
| WO 2013040251 | A2 | 21-03-2013 | US | 2013157272 A1 | 20-06-2013 |
| | | | US | 2018066316 A1 | 08-03-2018 |
| | | | WO | 2013040251 A2 | 21-03-2013 |
| WO 2014056626 | A1 | 17-04-2014 | CA | 2887920 A1 | 17-04-2014 |
| | | | EP | 2906953 A1 | 19-08-2015 |
| | | | HK | 1213640 A1 | 08-07-2016 |
| | | | JP | 2015534648 A | 03-12-2015 |
| | | | JP | 2018105876 A | 05-07-2018 |
| | | | US | 2015260721 A1 | 17-09-2015 |
| | | | WO | 2014056626 A1 | 17-04-2014 |
| WO 2015171736 | A2 | 12-11-2015 | EP | 3140426 A2 | 15-03-2017 |
| | | | US | 2017108502 A1 | 20-04-2017 |
| | | | WO | 2015171736 A2 | 12-11-2015 |
| WO 02103320 | A2 | 27-12-2002 | AT | 503023 T | 15-04-2011 |
| | | | AU | 2002316251 A1 | 02-01-2003 |
| | | | CA | 2451074 A1 | 27-12-2002 |
| | | | CY | 1111677 T1 | 07-10-2015 |
| | | | DK | 1410011 T3 | 18-07-2011 |
| | | | EP | 1410011 A2 | 21-04-2004 |
| | | | JP | 5237076 B2 | 17-07-2013 |
| | | | JP | 2005500832 A | 13-01-2005 |
| | | | JP | 2009131262 A | 18-06-2009 |
| | | | PT | 1410011 E | 25-07-2011 |
| | | | US | 2003224374 A1 | 04-12-2003 |
| | | | US | 2009157326 A1 | 18-06-2009 |
| | | | US | 2011301048 A1 | 08-12-2011 |
| | | | US | 2013116145 A1 | 09-05-2013 |
| | | | US | 2018305768 A1 | 25-10-2018 |
| | | | WO | 02103320 A2 | 27-12-2002 |
| US 2015018230 | A1 | 15-01-2015 | NONE | | |
| WO 2016045799 | A1 | 31-03-2016 | CA | 2962476 A1 | 31-03-2016 |
| | | | EP | 3198030 A1 | 02-08-2017 |
| | | | US | 2017260593 A1 | 14-09-2017 |
| | | | WO | 2016045799 A1 | 31-03-2016 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 19 30 5522

This annex lists the patent family members relating to the patent documents cited in the above-mentioned European search report.
The members are as contained in the European Patent Office EDP file on
The European Patent Office is in no way liable for these particulars which are merely given for the purpose of information.

17-10-2019

| Patent document cited in search report | Publication date | Patent family member(s) | Publication date |
|---|---|---|---|
| WO 2014056627 A1 | 17-04-2014 | CA 2886601 A1<br>EP 2906220 A1<br>HK 1213500 A1<br>JP 2015533130 A<br>US 2015290193 A1<br>WO 2014056627 A1 | 17-04-2014<br>19-08-2015<br>08-07-2016<br>19-11-2015<br>15-10-2015<br>17-04-2014 |
| WO 2015120069 A1 | 13-08-2015 | US 2016348182 A1<br>WO 2015120069 A1 | 01-12-2016<br>13-08-2015 |
| WO 2017180886 A1 | 19-10-2017 | AU 2017250663 A1<br>CA 3020628 A1<br>CN 109153993 A<br>EP 3443089 A1<br>JP 2019520037 A<br>KR 20180133497 A<br>US 2019161804 A1<br>WO 2017180886 A1 | 01-11-2018<br>19-10-2017<br>04-01-2019<br>20-02-2019<br>18-07-2019<br>14-12-2018<br>30-05-2019<br>19-10-2017 |
| WO 2017079632 A1 | 11-05-2017 | NONE | |
| WO 2013060739 A1 | 02-05-2013 | CA 2852020 A1<br>CN 104024436 A<br>DK 2771481 T3<br>EP 2771481 A1<br>ES 2689547 T3<br>JP 6049739 B2<br>JP 2015501151 A<br>SE 1150982 A1<br>US 2014243433 A1<br>US 2018080088 A1<br>WO 2013060739 A1 | 02-05-2013<br>03-09-2014<br>15-10-2018<br>03-09-2014<br>14-11-2018<br>21-12-2016<br>15-01-2015<br>25-04-2013<br>28-08-2014<br>22-03-2018<br>02-05-2013 |

EPO FORM P0459

For more details about this annex : see Official Journal of the European Patent Office, No. 12/82

## REFERENCES CITED IN THE DESCRIPTION

### Non-patent literature cited in the description

- **DERAMAUDT T ; RUSTGI AK ; MUTANT KRAS.** the initiation of pancreatic cancer. *Biochim Biophys Acta,* 25 November 2005, vol. 1756 (2), 97-101
- Digital multiplexed gene expression analysis using the NanoString nCounter system. **KULKARNI MM.** Curr Protoc Mol Biol. April 2011
- **MARIONI JC ; MASON CE ; MANE SM ; STEPHENS M ; GILAD Y.** RNA-seq: an assessment of technical reproducibility and comparison with gene expression arrays. *Genome Res.,* September 2008, vol. 18 (9), 1509-17
- **WANG Z ; GERSTEIN M ; SNYDER M.** RNA-Seq: a revolutionary tool for transcriptomics. *Nat Rev Genet.,* January 2009, vol. 10 (1), 57-63
- **STEINBOCK LJ ; RADENOVIC A.** The emergence of nanopores in next-generation sequencing. *Nanotechnology,* 2015, vol. 26 (7), 074003
- **DOBIN A ; DAVIS CA ; SCHLESINGER F ; DRENKOW J ; ZALESKI C ; JHA S, BATUT P ; CHAISSON M ; GINGERAS TR.** STAR: ultrafast universal RNA-seq aligner. *Bioinformatics,* 2013, vol. 29 (1), 15-21
- **LIAO Y ; SMYTH GK ; SHI W.** featureCounts: an efficient general-purpose program for assigning sequence reads to genomic features. *Bioinformatics,* 2014, vol. 30 (7), 923-30
- **DERAMAUDT T ; RUSTGI AK.** Mutant KRAS in the initiation of pancreatic cancer. *Biochim Biophys Acta,* 25 November 2005, vol. 1756 (2), 97-101
- **FELDMANN et al.** Molecular genetics of pancreatic intraepithelial neoplasia. *J Hepatobiliary Pancreat Surg.,* 2007, vol. 14 (3), 224-32
- **IOVANNA et al.** Current knowledge on pancreatic cancer. *Front Oncol.,* 2012, vol. 2 (6
- **YING et al.** Genetics and biology of pancreatic ductal adenocarcinoma. *Genes Dev.,* 2016, vol. 30 (4), 355-85
- **RYAN CARR ; MARTIN E. FERNANDEZ-ZAPICO.** Pancreatic cancer microenvironment, to target or not to target. *EMBO Mol Med.,* February 2016, vol. 8 (2), 80-82
- **VENU G PILLARISETTY.** The pancreatic cancer microenvironment: an immunologic battleground. *OncoImmunology,* 01 August 2014, vol. 3 (8), e950171

- **J. IOVANNA ; CLOSA.** Factors released by the tumor far microenvironment are decisive for pancreatic adenocarcinoma development and progression. *Oncoimmunology,* 08 August 2017, vol. 6, 11
- **LECA, J. et al.** Cancer-associated fibroblast-derived annexin A6+ extracellular vesicles support pancreatic cancer aggressiveness. *J Clin Invest.,* 2016, vol. 126 (11), 4140-4156
- **HA BURRIS et al.** Improvements in survival and clinical benefit with gemcitabine as first-line therapy for patients with advanced pancreas cancer: a randomized trial. *J Clin Oncol.,* June 1997, vol. 15 (6), 2403-13
- **THIERRY CONROY et al.** FOLFIRINOX versus Gemcitabine for Metastatic Pancreatic Cancer. *New England Journal of Medicine,* 2011, vol. 364, 1817-1825
- **NICOLLE et al.** Pancreatic Adenocarcinoma Therapeutic Targets Revealed by Tumor-Stroma Cross-Talk Analyses in Patient-Derived Xenografts. *Cell Rep.,* 28 November 2017, vol. 21 (9), 2458-2470
- **BAILEY P et al.** Australian Pancreatic Cancer Genome Initiative. Genomic analyses identify molecular subtypes of pancreatic cancer. *Nature,* 2016, vol. 531, 47-52
- **GWEN LOMBERK et al.** Distinct epigenetic landscapes underlie the pathobiology of pancreatic cancer subtypes. *NATURE COMMUNICATIONS,* 2018, vol. 9, 1978, www.nature.com/naturecommunications
- **JIRI LENZ et al.** Clinicopathological correlations of nestin expression in surgically resectable pancreatic cancer including an analysis of perineural invasion; Journal of gastrointestinal and liver diseases. *JGLD,* 2011, vol. 20 (4), 389-396
- **P. VILMANN et al.** Endoscopic ultrasonography with guided fine needle aspiration biopsy in pancreatic disease. *Gastrointest Endosc.,* March 1992, vol. 38 (2), 172-3
- **PAULINE DUCONSEIL et al.** Transcriptomic Analysis Predicts Survival and Sensitivity to Anticancer Drugs of Patients with a Pancreatic Adenocarcinoma. *The American Journal of Pathology,* April 2015, vol. 185 (4
- **KULKARNI MM.** Digital multiplexed gene expression analysis using the NanoString nCounter system. *Curr Protoc Mol Biol.,* April 2011

- **DOBIN A ; DAVIS CA ; SCHLESINGER F ; DRENKOW J ; ZALESKI C ; JHA S ; BATUT P ; CHAISSON M ; GINGERAS TR.** STAR: ultrafast universal RNA-seq aligner. *Bioinformatics,* 01 January 2013, vol. 29 (1), 15-21
- **BULLARD et al.** Evaluation of statistical methods for normalization and differential expression in mRNA-Seq experiments. *BMC Bioinformatics,* 2010, vol. 11 (94, http://www.biomedcentral.com/1471-2105/11/94
- **MOFFITT RA ; MARAYATI R ; FLATE EL et al.** Virtual microdissection identifies distinct tumor- and stroma-specific subtypes of pancreatic ductal adenocarcinoma. *Nat Genet,* 2015, vol. 47, 1168-1178
- **MAURER HC ; HOLMSTROM SR ; HE J et al.** Experimental microdissection enables functional harmonisation of pancreatic cancer subtypes. *Gut,* 2019